# EUROPEAN PATENT APPLICATION

(11) **EP 4 151 634 A1**
(43) Date of publication of application: **22.03.2023**
(21) Application number: 21804286.9
(22) Date of filing: 08.05.2021
(51) Int. Cl.: C07D 413/14, C07D 471/04, C07D 207/04, A61K 31/33, A61P 35/00, A61P 37/00

(54) **PREPARATION OF BIARYL RING-LINKED AROMATIC HETEROCYCLIC DERIVATIVE AS IMMUNOMODULATOR AND USE THEREOF**

(30) Priority: 11.05.2020 CN 202010394259
(71) Applicant: Shanghai Longwood Biopharmaceuticals Co., Ltd., Shanghai 201108 (CN)
(72) Inventor: WANG, Zhe, Shanghai 201108 (CN); QIAN, Anran, Shanghai 201108 (CN); LI, Deliang, Shanghai 201108 (CN)
(74) Representative: Lavoix
(86) International application number: PCT/CN2021/092482
(87) International publication number: WO 2021/228000

(57) **Abstract**

The preparation of an aromatic heterocyclic derivative as an immunomodulator and the use thereof. Specifically, provided is a compound as represented by formula I below, or an optical isomer, a hydrate, a solvate, or a pharmaceutically acceptable salt thereof, wherein the definition of each group is as described in the description; and the compound of formula I can be used to treat diseases associated with the PD-1/PD-L1 signaling pathway.

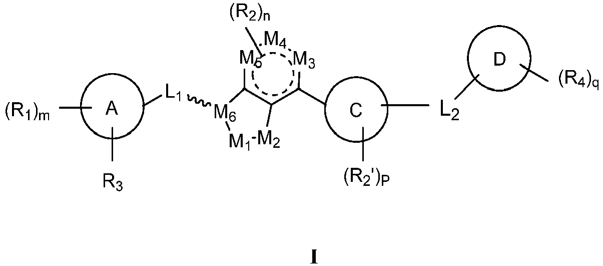

## Description

### TECHNICAL FIELD

The present invention relates to the field of small molecule drugs, and specifically, the present invention provides a small molecule compound that can be used to treat diseases related to PD-1/PD-L1 signaling pathway.

### BACKGROUND OF THE INVENTION

The immune system plays a vital role in controlling and curing many diseases, such as various cancers, diseases caused by viruses, etc. But the cancer cells can evade or inhibit the immune system in some way to proliferate rapidly. One way is to alter the activator molecules and inhibitory molecules expressed on immune cells. Blocking inhibitory immune checkpoints, such as PD-1, has been proven to be a very effective approach to suppressing cancer cells.

PD-1 is programmed cell death protein-1, also known as CD279. It is mainly expressed in activated T cells and B cells, and its function is to inhibit the activation of cells, which is a normal homeostatic mechanism of the immune system, because excessive T/B cell activation can cause autoimmune diseases, PD-1 is a protective wall of our body. PD-1 is a type I transmembrane glycoprotein composed of 268 amino acids, and its structure mainly includes the outer immunoglobulin variable domain, the hydrophobic transmembrane domain and the intracellular domain. The intracellular domain contains two phosphorylation sites, and they are located in the immunoreceptor tyrosine inhibitory motif and the immunoreceptor tyrosine switching motif, respectively, which also proves that PD-1 can negatively regulate T cell receptor-mediated signal. PD-1 has two ligands, PD-L1 and PD-L2, which differ in expression way. PD-L1 is up-regulated in a variety of tumor cells, and it binds to PD-1 on T cells, inhibits T cell proliferation and activation, makes T cells in a state of inactivation, and ultimately induces immune escape.

PD-1/PD-L1 plays an inverse immunomodulatory role. When PD-1 binds to PD-L1, it can cause tyrosine polyphosphorylation in the immunoreceptor tyrosine switch motif domain of T cells, and the phosphorylated tyrosine can bind to the phosphatase protein tyrosinase 2 and protein tyrosinase 1, which can impede the activation of extracellular signal-regulated kinase and also block the activation of phosphatidylinositol 3-kinase (PI3K) and serine-threonine protein kinase (Akt), thereby inhibiting T lymphocyte proliferation and the secretion of related cytokines. The PD-1/PD-L1 signal inhibits the activation and proliferation of T cells, and at the same time, it can also induce the secretion of cytokines interleukin 2, interferon γ and IL-10. In addition, PD-1/PD-L1 signaling also has a similar immune function on B cells. After PD-1 binds to B cell antigen receptors, the PD-1 cytoplasmic domain interacts with tyrosinase containing a protein tyrosinase 2 binding site, thereby hindering B cell activation.

The immunotherapy based on PD-1/PD-L1 is a new generation of immunotherapy that has attracted much attention. In recent years, a series of surprising findings have confirmed that PD-1/PD-L1 inhibitors have strong antitumor activity against a variety of tumors. Currently available PD-1/PD-L1 antibody inhibitors include BMS's Ninolumab, Merck's Lambrolizumab and Roche's Atezolizumab. In addition, there are many PD-1/PD-L1 antibody inhibitors in development, including CureTech's Pidilizumab, GSK's AMP-224 and AstraZeneca's MEDI-4736.

Although tumor immunotherapy is considered to be a new generation of revolution in cancer treatment after targeted therapy, the PD-1 monoclonal antibodies currently on the market and under development have their own shortcomings. They can only be administered by injection and cannot be taken orally. They are unstable in the body, are easily decomposed by proteases, and are prone to immune cross-reaction. Moreover, it is difficult to purify and the production cost is high. Therefore, small molecule inhibitor of PD-1/PD-L1 interaction is a better option for tumor immunotherapy.

In summary, there is an urgent need in the art to develop novel small-molecule inhibitor of PD-1/PD-L1 interaction.

### SUMMARY OF THE INVENTION

The object of the present invention is to provide a novel small-molecule inhibitor of PD-1/PD-L1 interaction.

The first aspect of the invention provides a compound shown in Formula I below, or optical isomers, cis-trans isomers, hydrates, solvates thereof, or pharmaceutically acceptable salts thereof: 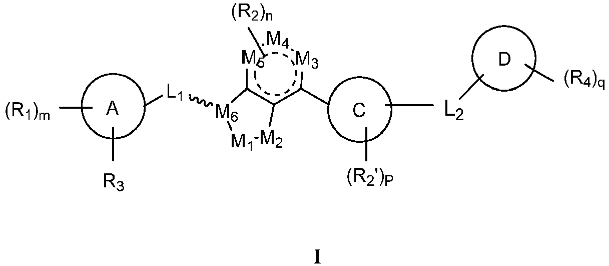
wherein, n, m, p and q are each independently selected from 0, 1, 2, 3 or 4;
L₁ and L₂ are each independently selected from the group consisting of chemical bond, substituted or unsubstituted C₁-C₄ alkylene, substituted or unsubstituted C₂-C₄ alkenylene, substituted or unsubstituted C₂-C₄ alkenyl, -S-, -O-, substituted or unsubstituted -NH-, -S(O)-, -S(O)₂-, substituted or unsubstituted -NHC(O)NH-, 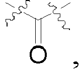 substituted or unsubstituted 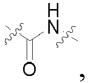 substituted or unsubstituted 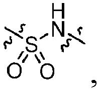 substituted or unsubstituted 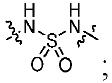
M₁ and M₂ are each independently selected from the group consisting of C(R₆)₂, NR₆, O, S, SO, SO₂; wherein, R₆ is selected from the group consisting of H, chlorine, bromine, fluorine, iodine, cyano, hydroxyl, nitro, NR_{f}, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, -C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, substituted or unsubstituted C₂-C₆ alkenyl, substituted or unsubstituted C₂-C₆ alkynyl, -C(=O)-substituted or unsubstituted C₂-C₆ alkenyl, and -C(=O)-substituted or unsubstituted C₂-C₆ alkynyl;
M₃, M₄ and M₅ are each independently selected from the group consisting of chemical bond, CR₆, N, NR₆, O, S, SO and SO₂;
M₆ is selected from the group consisting of CR₆, N;
and the 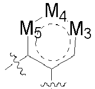 is aromatic ring; 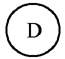
is a group having the following structure: 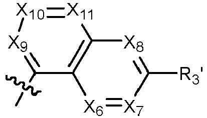 or 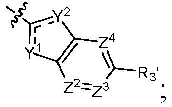
wherein,
X⁶, X⁷, X⁸, X⁹, X¹⁰, X¹¹, Z², Z³, and Z⁴ are each independently selected from the group consisting of N, N-O, CRₐ; wherein, the Rₐ is selected from the group consisting of H, chlorine, bromine, fluorine, iodine, cyano, hydroxyl, nitro, NR_{f}, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, -C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, substituted or unsubstituted C₂-C₆ alkenyl, substituted or unsubstituted C₂-C₆ alkynyl, -C(=O)-substituted or unsubstituted C₂-C₆ alkenyl, and -C(=O)-substituted or unsubstituted C₂-C₆ alkynyl;
Y¹ and Y² are each independently selected from the group consisting of CH, CH₂, NH, N, N-O, CF, CRₐ, O, S, SO or SO₂;
-̅ -̅ -̅ is single bond or double bond;
and 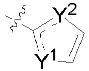 is an aromatic or non-aromatic fragment; 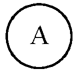
is selected from the group consisting of substituted or unsubstituted 5-12 membered heteroaryl, substituted or unsubstituted C₆-C₁₀ aryl, substituted or unsubstituted 5-12 membered heterocyclyl, substituted or unsubstituted 5-12 membered C₅-C₁₂ ring group, wherein the 5-12 membered heteroaryl and 5-12 membered heterocyclyl have 1-4 heteroatoms selected from B, P, N, O, S, wherein P, N, O as ring atoms can be oxygenated and one or more cyclic carbon atoms can be replaced with carbonyl; 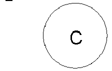
is a divalent group formed by a ring selected from the group consisting of 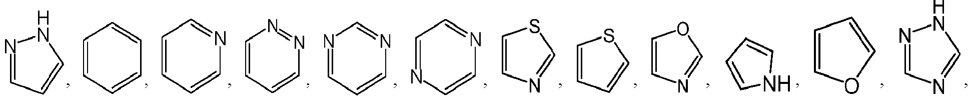 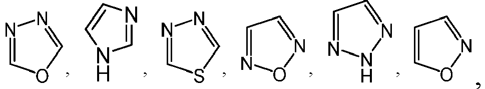 wherein bonding position of the ring can be N or C;
R₁, R₂, R₂ and R₄ are each independently selected from the group consisting of H, halogen, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted C₃-C₈ cycloalkyl, oxy (i.e.=O), =NR_{f}, -CN, hydroxyl, NR_{d}Rₑ (e.g. amino), substituted or unsubstituted C₁-C₆ amino, substituted or unsubstituted -(C₁-C₆ alkylene)-NH-(C₁-C₆ alkylene), carboxyl, substituted or unsubstituted C₆-C₁₀ aryl, substituted or unsubstituted 5-12 membered heteroaryl with 1-3 heteroatoms, substituted or unsubstituted 5-12 membered heterocyclyl with 1-4 heteroatoms, substituted or unsubstituted 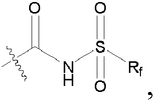 substituted or unsubstituted 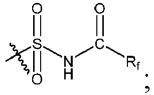 or -(L₁ₐ)ᵣ-(L₂ₐ)ₛ-(L₃ₐ)ₛ-;
R₃ and R₃'are 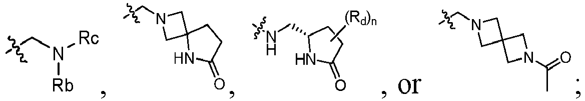 wherein R_{b}, R_{c} and R_{d} are each independently selected from the group consisting of H, substituted or unsubstituted C₁-C₈ alkyl; or R_{b} and R_{c} together with the adjacent N atom form substituted or unsubstituted 5-10 membered heterocyclyl with 1-3 heteroatoms selected from N, S and O;
each L₁ₐ is each independently selected from the group consisting of chemical bond, substituted or unsubstituted C₁-C₇ alkylene, substituted or unsubstituted C₂-C₄ alkenylene, substituted or unsubstituted C₂-C₄ alkynylene, -S-, -O-, substituted or unsubstituted -NH-, -S(O)-, -S(O)₂-;
L₂ₐ is selected from the group consisting of substituted or unsubstituted C₆-C₁₂ arylene, substituted or unsubstituted 5-12 membered heteroarylene with 1-3 heteroatoms, substituted or unsubstituted C₃-C₈ cycloalkylene, substituted or unsubstituted 5-10 membered heterocyclylene with 1-3 heteroatoms;
L₃ₐ is selected from the group consisting of H, substituted or unsubstituted C₁-C₁₀ alkyl, C₁-C₁₀ aryl, -CN, hydroxyl, amino, carboxyl, -CO-NH-SO₂-R_{g}, -NH-SO₂-R_{g}, -SO₂-NH-CO-R_{g};
R_{d}, Rₑ and R_{g} are each independently selected from the group consisting of H, substituted or unsubstituted C₁-C₆ alkyl, substituted or unsubstituted C₃-C₁₀ cycloalkyl, substituted or unsubstituted C₆-C₁₀ aryl;
R_{f} is selected from the group consisting of H, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, cyano, -C(=O)-NR_{d}Rₑ, - C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, -C(=O)-substituted or unsubstituted C₂-C₆ alkynyl; unless otherwise specified, the "substituted" means being substituted by one or more (such as 2, 3, 4, etc.) substituents selected from the group consisting of halogen (including but not limited to F, Cl, Br), -CH₂Cl, -CHCl₂, -CCl₃, -CH₂F, -CHF₂, -CF₃, oxo, -CN, hydroxyl, amino, C₁-C₆ alkyl amino, carboxyl, -NHAc, or substituted or unsubstituted groups which are selected from C₁-C₆ alkyl, C₁-C₆ alkoxy, C₆-C₁₀ aryl, C₃-C₈ cycloalkyl, halogenated C₆-C₁₀ aryl, 5-10 membered heteroaryl with 1-3 heteroatoms selected from N, S and O, and 5-10 membered heterocyclyl with 1-3 heteroatoms selected from N, S and O, and the substituent of which is selected from halogen, hydroxyl, carboxyl, cyano, C₁-C₆ alkoxy, and C₁-C₆ alkylamino;
among the above formula, any of the heteroatoms is selected from the group consisting of B, P, N, S and O.

In another preferred embodiment, R₁, R₂, R₂' and R₄ are each independently selected from the group consisting of H, halogen, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted C₃-C₈ cycloalkyl, oxy (i.e.=O), =NR_{f}, -CN, hydroxyl, NR_{d}Rₑ (e.g. amino), substituted or unsubstituted C₁-C₆ amino, substituted or unsubstituted -(C₁-C₆ alkylene)-NH-(C₁-C₆ alkylene), carboxyl, substituted or unsubstituted C₆-C₁₀ aryl, substituted or unsubstituted 5-12-membered heteroaryl with 1-3 heteroatoms, substituted or unsubstituted 5-12-membered heterocyclyl with 1-4 heteroatoms substituted or unsubstituted 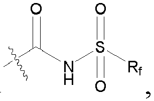 substituted or unsubstituted 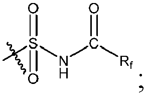 or -(L₁ₐ)ᵣ-(L₂ₐ)ₛ-(L₃ₐ)ₛ-.

In another preferred embodiment, the ring 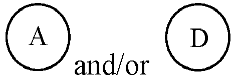 have substituents as shown in Formula IV below: 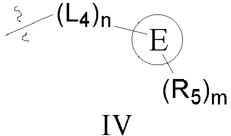
wherein, each L₄ is independently selected from the group consisting of substituted or unsubstituted C₁-C₄ alkylene, -S-, -O-, -NRₐ-, -S(O)-, -S(O)₂-; preferably substituted or unsubstituted C₁-C₄ alkylene, provided that the structure formed by each L₄ is chemically stable; 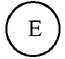
is selected from the group consisting of substituted or unsubstituted C₅-C₁₀ cycloalkyl, substituted or unsubstituted 3-10 membered heterocyclyl with 1-3 heteroatoms selected from B, P, N, S and O; preferably, 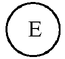 is nitrogen-containing 3-8 membered heterocyclyl;
each R₅ is each independently selected from the group consisting of substituted or unsubstituted C₁-C₆ alkyl, -CN, hydroxyl, amino and carboxyl; wherein, the substituent is selected from the group consisting of halogen, hydroxyl, carboxyl, cyano, C₁-C₆ alkoxy.

In another preferred embodiment, the compound of formula I has the structure shown in the following formula:

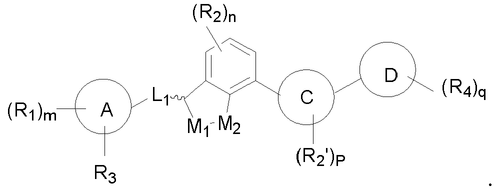

In another preferred embodiment, 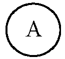 is the structure shown in the following formula: 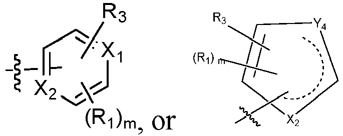 wherein, X₁, X₂ and Y₄ are each independently selected from CH (the CH can be substituted by R₁ or R₃), N, O, S and NH (the NH can be substituted by R₁ or R₃).

In another preferred embodiment, M₁ and M₂ are each independently CH₂.

In another preferred embodiment, the compound has the structure shown in Formula I-a or I-b as follows: 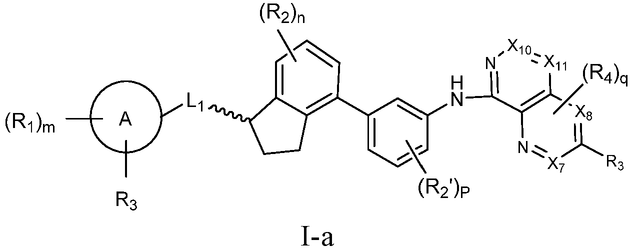 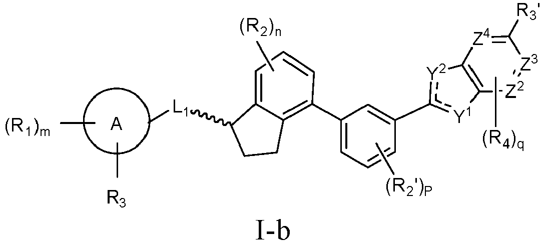 wherein,
R₃ and R₃' are each independently 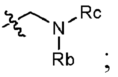 wherein, R_{b} and R_{c} together with adjacent N atom form substituted or unsubstituted 5-10 membered heterocyclyl (preferably 5-7 membered heterocyclyl) with 1-3 heteroatoms selected from N, S and O;
other groups are defined as described above.

In another preferred embodiment, R₃ and R₃' are each independently

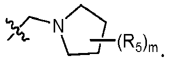

In another preferred embodiment, the compound is selected from the group consisting of:

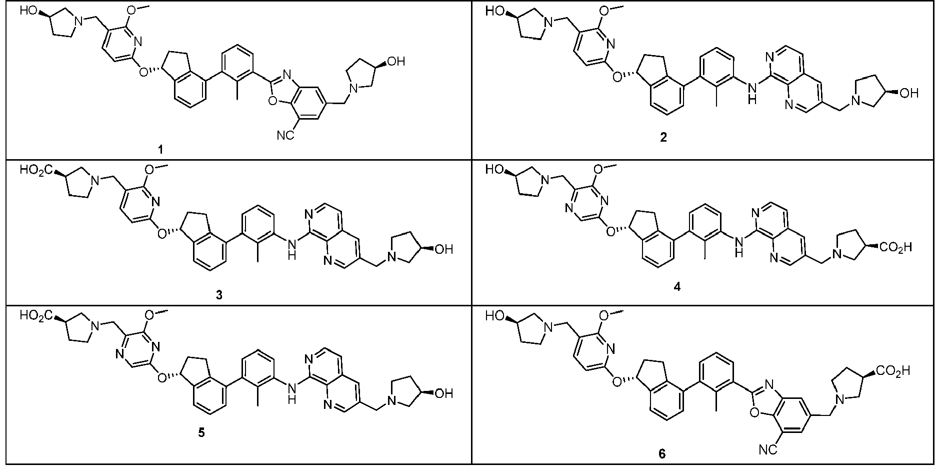

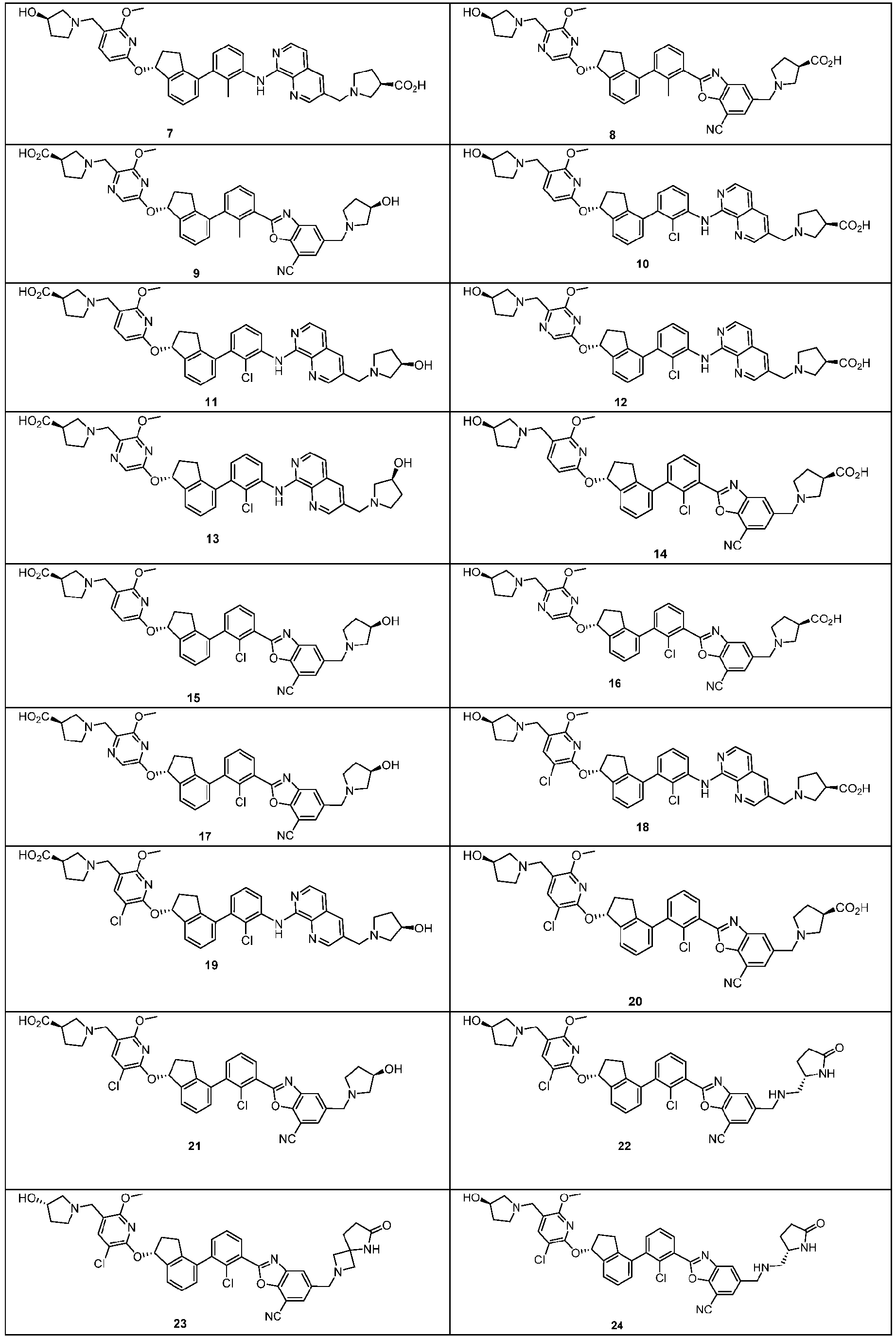

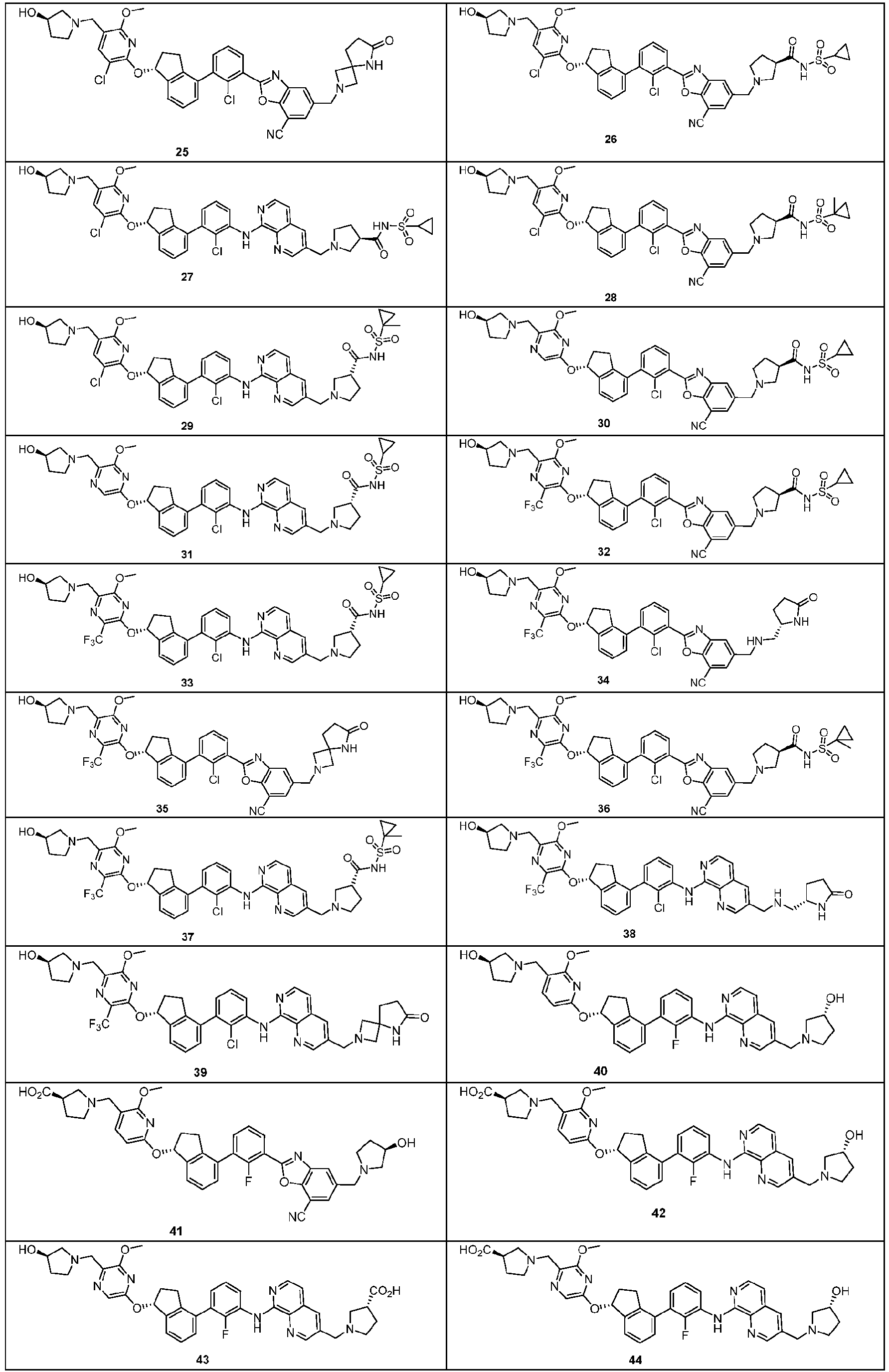

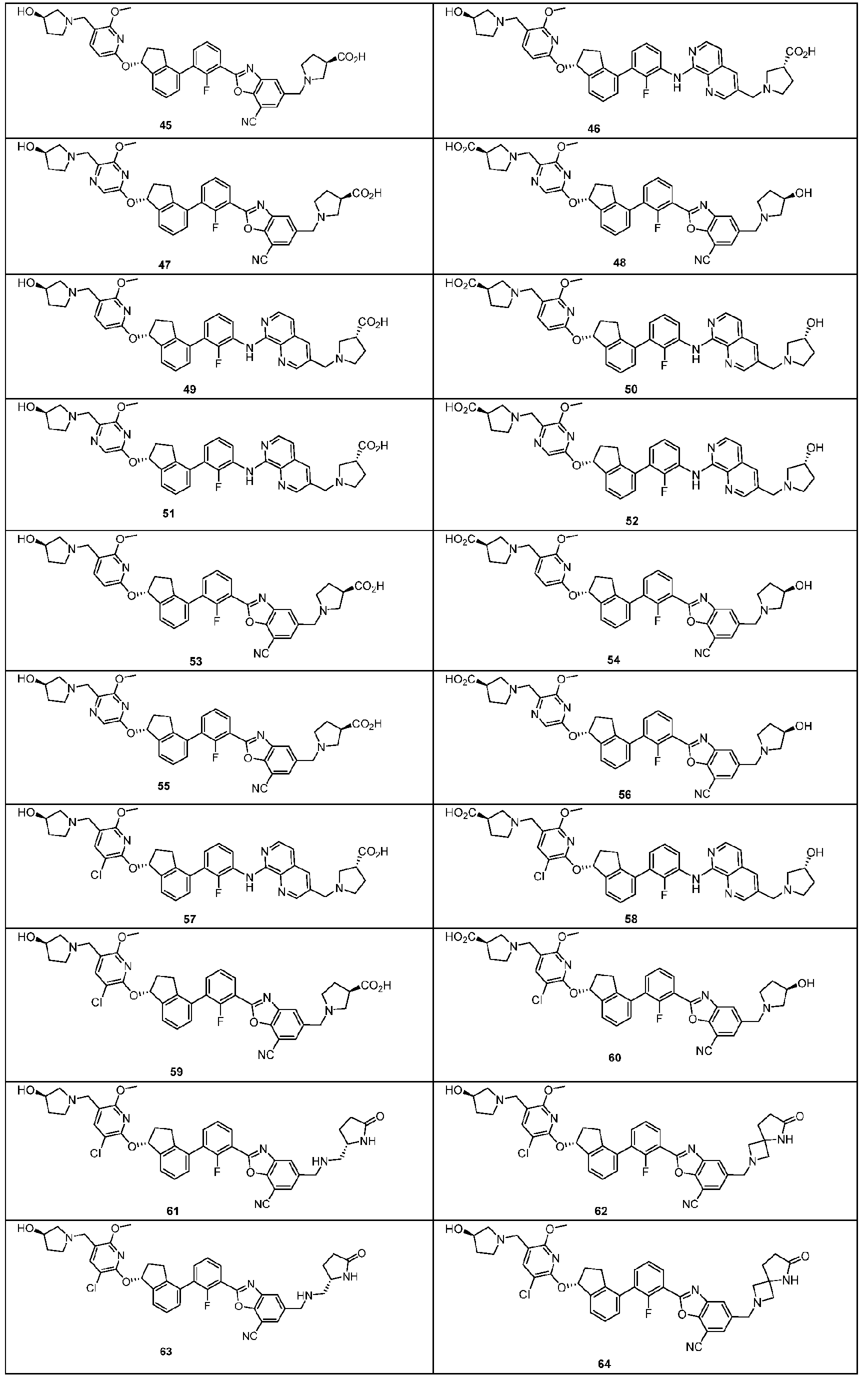

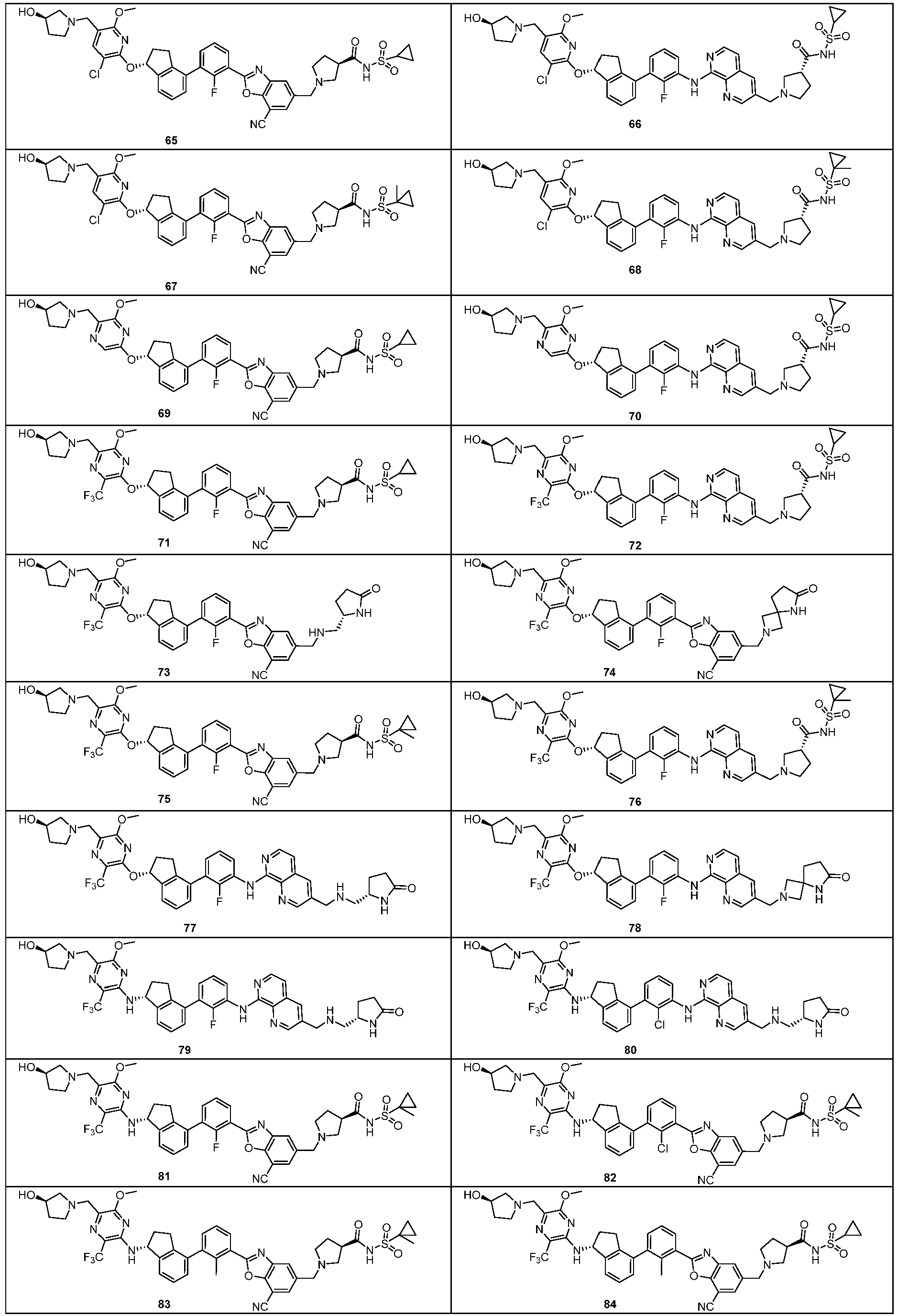

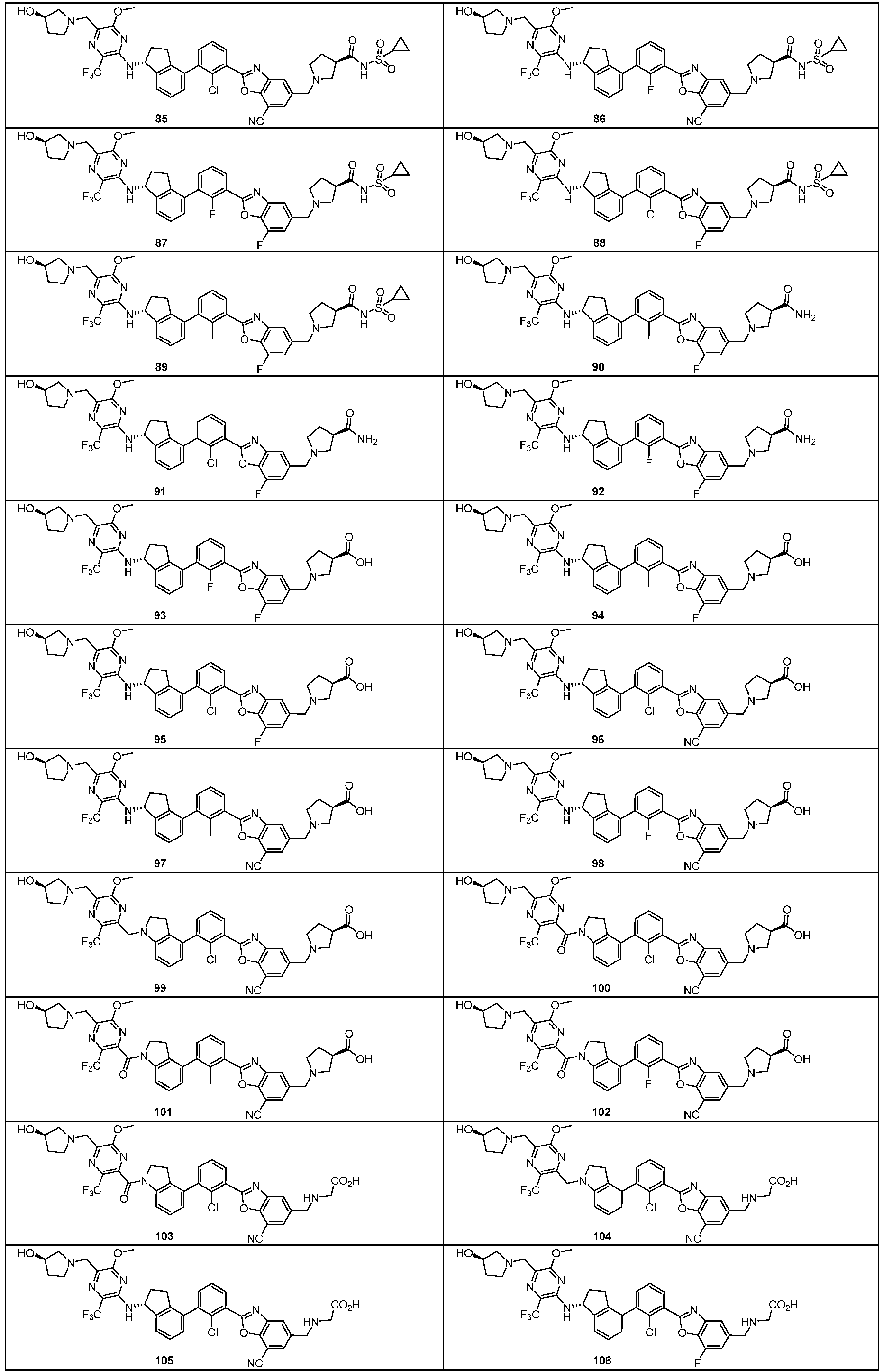

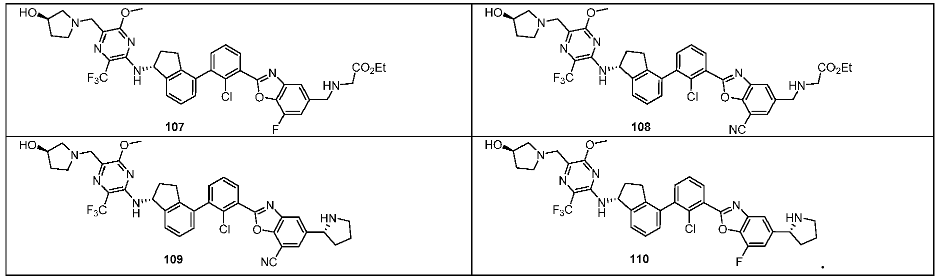

The second aspect of the invention provides a method for preparing compounds of formula I as described in the first aspect of the invention, and the method comprises the following steps: 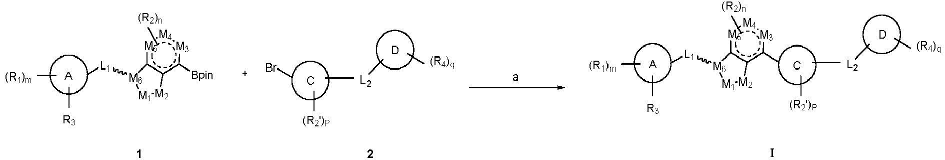
(a) providing the target product I by Suzuki coupling reaction catalyzed by appropriate palladium catalyst with intermediates **1** and **2** as raw materials;
wherein, the definition of each group is as described in the first aspect of the invention.

In another preferred embodiment, the preparation method of intermediate **1** is as follows: 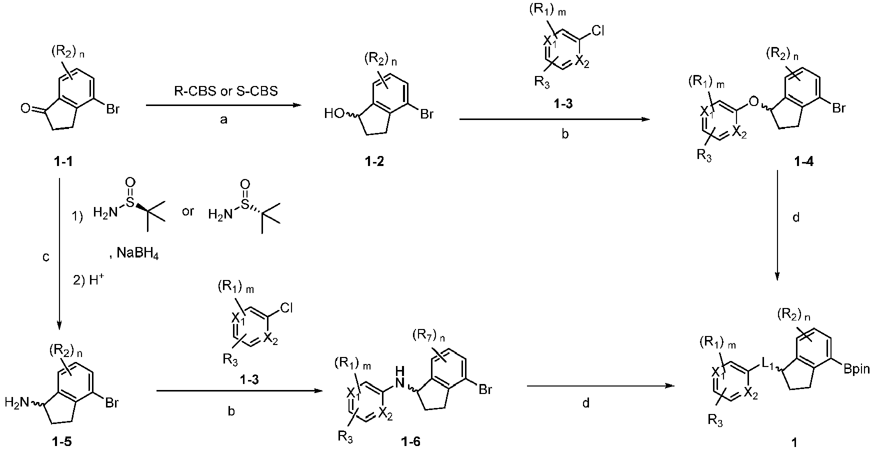
providing chiral alcohol **1-2** with compound **1-1** as raw material with chiral adjuvant (e.g., R/S-CBS) and reducing agent (e.g., borane);
(b) obtaining the intermediate **1-4** (or **1-6**) by coupling reaction (such as Suzuki, Buchwald, etc.) of **1-2** (or **1-5**) and **1-3** with suitable palladium catalyst and ligand;
(c) providing protected chiral amino compound by reaction of compound **1-1** and R/S-tert-butyl sulfonimide under Lewis acid (such as ethyl tetrasticotitanate) and reducing reagent (such as lithium aluminum hydride, sodium borohydride, etc.), and then removing the protecting group under acidic condition to obtain chiral amino compound **1-5**;
(d) obtaining the intermediate **1** by Suzuki coupling reaction between **1-4** (or **1-6**) and bis(pinacolato)diboron under suitable palladium catalyst and ligand.

In another preferred embodiment, the preparation method of intermediate **2** is as follows:
method 1: 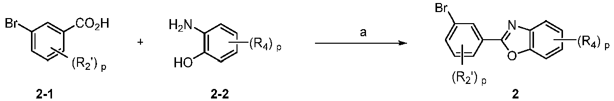 providing intermediate **2** by reacting compounds **2-1** and **2-2** under dehydrating agent (such as concentrated sulfuric acid, PPA, etc.);
method 2: 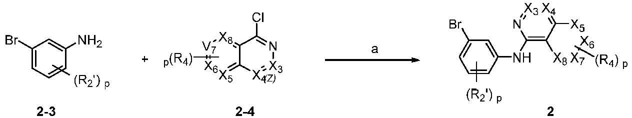 providing intermediate 2 by nucleophilic substitution with compounds 2-3 and 2-4 as raw materials under acidic or alkaline condition.

The third aspect of the present invention provides a pharmaceutical composition, which comprises (1) the compound according to the first aspect of the present invention or stereoisomers or tautomers thereof, or pharmaceutically acceptable salts, hydrates or solvates thereof; (2) pharmaceutically acceptable carriers.

In another preferred embodiment, the pharmaceutical composition is used to treat a disease selected from the group consisting of cancer, infectious disease, and autoimmune disease.

In another preferred embodiment, the cancer is selected from the group consisting of pancreatic cancer, bladder cancer, colorectal cancer, breast cancer, prostate cancer, kidney cancer, hepatocellular carcinoma, lung cancer, ovary cancer, cervical cancer, stomach cancer, esophageal cancer, melanoma, neuroendocrine cancer, central nervous system cancer, brain cancer, bone cancer, soft tissue sarcoma, non-small cell lung cancer, small cell lung cancer or colon cancer, skin cancer, lung cancer, urinary system tumor, blood tumor, glioma, digestive system tumor, reproductive system tumor, lymphoma, nervous system tumor, brain tumor, and head and neck cancer.

In another preferred embodiment, the cancer is selected from the group consisting of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disorder (MPD), chronic myeloid leukemia (CML), multiple myeloma (MM), non-hodgkin lymphoma (NHL), mantle cell lymphoma (MCL), follicular lymphoma, Waldestrom macroglobulinemia (WM), T-cell lymphoma, B-cell lymphoma, and diffuse large B-cell lymphoma (DLBCL).

In another preferred embodiment, the infectious disease is selected from bacterial infection and viral infection.

In another preferred embodiment, the autoimmune disease is selected from the group consisting of organ-specific autoimmune disease and systemic autoimmune disease.

In another preferred embodiment, the organ-specific autoimmune diseases include chronic lymphocytic thyroiditis, hyperthyroidism, insulin-dependent diabetes mellitus, myasthenia gravis, ulcerative colitis, pernicious anemia with chronic atrophic gastritis, pulmonary hemorrhagic nephritic syndrome, primary biliary cirrhosis, multiple cerebral sclerosis, and acute idiopathic polyneuritis.

In another preferred embodiment, the systemic autoimmune diseases include rheumatoid arthritis, systemic lupus erythematosus, systemic vasculitis, scleroderma, pemphigus, dermatomyositis, mixed connective tissue disease, and autoimmune hemolytic anemia.

In another preferred embodiment, the pharmaceutical composition is also used to improve T cell function in a patient with chronic hepatitis B (CHB).

In another preferred embodiment, the inhibitor further comprises at least one therapeutic agent selected from the group consisting of nivolumab, pembrolizumab, atezolizumab and ipilimumab.

The fourth aspect of the present invention provides a use of the compound according to the first aspect of the present invention or stereoisomers or tautomers thereof, or pharmaceutically acceptable salts, hydrates or solvates thereof, or a pharmaceutical composition according to the third aspect of the present invention, for the preparation of a pharmaceutical composition for preventing and/or treating diseases related to the activity or expression of PD-1/PD-L1.

The fifth aspect of the present invention provides a PD-1/PD-L1 inhibitor, the inhibitor comprises the compound according to the first aspect of the present invention, or stereoisomers or tautomers thereof, or pharmaceutically acceptable salts, hydrates or solvates thereof.

The sixth aspect of the present invention provides a method for inhibiting the interaction of PD-1/PD-L1 *in vitro,* which comprises the steps of: contacting the compound according to the first aspect of the present invention, or stereoisomers or tautomers thereof, or pharmaceutically acceptable salts, hydrates or solvates thereof with a PD-L1 protein.

It should be understood that, within the scope of the present invention, the above technical features of the present invention and the technical features specifically described in the following descriptions (such as the examples) can be combined with each other to form a new or preferred technical solution. Due to space limitations, they will not be repeated herein.

### DETAILED DESCRIPTION OF THE INVENTION

After extensive and intensive research, the present inventors discovered a class of PD-1/PD-L1 interaction inhibitors with excellent inhibitory effect. The present invention has been completed on this basis.

### Definitions

As used herein, the term "alkyl" includes straight or branched alkyl groups. For example, C₁-C₈ alkyl refers to straight or branched alkyls having from 1-8 carbon atoms, such as methyl, ethyl, propyl, isopropyl, butyl, isobutyl, tert-butyl, and the like.

As used herein, the term "alkenyl" includes straight or branched alkenyl groups. For example, C₂-C₆ alkenyl refers to straight or branched alkenyl groups having 2-6 carbon atoms, such as vinyl, allyl, 1-propenyl, isopropenyl, 1-butenyl, 2-butenyl, and the like.

As used herein, the term "alkynyl" includes straight or branched alkynyl groups. For example, "C₂-C₆ alkynyl" refers to straight or branched alkynyl group having 2-6 carbon atoms, such as ethynyl, propynyl, butynyl, and the like.

As used herein, the term "C₃-C₁₀ cycloalkyl" refers to cycloalkyl groups having 3 to 10 carbon atoms. It may be a monocyclic ring, such as cyclopropyl, cyclobutyl, cyclopentyl, cyclohexyl, and the like. It may also be in bicyclic form, such as bridged or spiro ring form.

As used herein, the term "C₁-C₈ alkylamino" refers to amine groups substituted with C₁-C₈ alkyl group, which may be mono- or di-substituted; for example, methylamino, ethylamino, propylamino, isopropylamine, butylamine, isobutylamine, tert-butylamine, dimethylamine, diethylamine, dipropylamine, diisopropylamine, dibutylamine, diisobutylamine, di-tert-butylamine, and the like.

As used herein, the term "C₁-C₈ alkoxy" refers to straight or branched alkoxy groups having 1-8 carbon atoms; for example, methoxy, ethoxy, propoxy, isopropoxy, butoxy, isobutoxy, tert-butoxy, and the like.

As used herein, the term "3-10 membered heterocycloalkyl having 1-3 heteroatoms selected from the group consisting of N, S and O" refers to a saturated or partially saturated cyclic group having 3-10 atoms, wherein 1-3 atoms are heteroatoms selected from the group consisting of N, S and O. It may be a monocyclic ring or in a bicyclic form, such as bridged or spiro ring form. Specific examples may be oxetane, azetidine, tetrahydro-2H-pyranyl, piperidinyl, tetrahydrofuranyl, morpholinyl, pyrrolidinyl, and the like.

As used herein, the term "C₆-C₁₀ aryl" refers to aryl groups having 6 to 10 carbon atoms, such as phenyl, naphthyl, and the like.

As used herein, the term "5-10 membered heteroaryl having 1-3 heteroatoms selected from the group consisitng of N, S and O" refers to cyclic aromatic groups having 5-10 atoms, of which 1-3 atoms are selected from the group consisting of N, S and O. It may be a monocyclic ring or in a fused ring form. Specific examples may be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazyl, furyl, thienyl, isoxazolyl, thiazolyl, oxazolyl, and the like.

Unless otherwise specified as "substituted or unsubstituted", the group described in the present invention can be substituted by a substituent selected from the group consisting of halogen, nitrile, nitro, hydroxy, amino, C₁-C₆ alkyl-amine, C₁-C₆ alkyl, C₂-C₆ alkenyl, C₂-C₆ alkynyl, C₁-C₆ alkoxy, halogenated C₁-C₆ alkyl, halogenated C₂-C₆ alkenyl, halogenated C₂-C₆ alkynyl, halogenated C₁-C₆ alkoxy, allyl, benzyl, C₆-C₁₂ aryl, C₁-C₆ alkoxy-C₁-C₆ alkyl, C₁-C₆ alkoxy-carbonyl, phenoxycarbonyl, C₂-C₆ alkynyl-carbonyl, C₂-C₆ alkenyl-carbonyl, C₃-C₆ cycloalkyl-carbonyl, C₁-C₆ alkylsulfonyl, etc.

As used herein, "halogen" or "halogen atom" refers to F, Cl, Br, and I. More preferably, the halogen or halogen atom is selected from F, Cl and Br. "Halogenated" means substituted with an atom selected from F, Cl, Br, and I.

Unless otherwise specified, the structural formula described herein are intended to include all isomeric forms (such as enantiomeric, diastereomeric, and geometric isomers (or conformational isomers)): for example, R, S configuration of compounds with asymmetrical centers, (Z), (E) isomers of double bonds, etc. Therefore, the single stereochemical isomers or enantiomers, diastereomers or geometric isomers (or conformers) of the compounds of the invention, or mixtures thereof all fall within the scope of the invention.

As used herein, the term "tautomer" means that structural isomers having different energies can exceed the low energy barrier and thereby transform between each other. For example, proton tautomers (proton shift) include interconversion by proton transfer, such as 1H-carbazole and 2H-carbazole. Valence tautomers include interconversion through some bonding electron recombination.

As used herein, the term "solvate" refers to a complex formed by coordinating a compound of the invention with a solvent molecule in specific proportion.

As used herein, the term "hydrate" refers to a complex formed by coordinating a compound of the invention with water.

It should be understood that, in this context, the definition of each group is intended to form a chemically stable structure.

### Active ingredient

As used herein, "compounds of the present invention" refers to compounds of formula I, and also includes various crystalline forms, pharmaceutically acceptable salts, hydrates or solvates of the compounds of formula I.

Preferred compounds of the present invention include compounds 1-360 (including various R- and/or S-configuration stereoisomers, and/or E-/Z- cis-trans isomers of each compound).

In another preferred embodiment, the pharmaceutically acceptable salts include salts formed by combining with inorganic acids, organic acids, alkali metal ions, alkaline earth metal ions or organic bases capable of providing physiologically acceptable cations and ammonium salts.

In another preferred embodiment, the inorganic acids are selected from hydrochloric acid, hydrobromic acid, phosphoric acid or sulfuric acid; the organic acids are selected from methanesulfonic acid, p-toluenesulfonic acid, trifluoroacetic acid, medlaric acid, maleic acid, tartaric acid, fumaric acid, citric acid or lactic acid; the alkali metal ions are selected from lithium ion, sodium ion, potassium ion; the alkaline earth metal ions are selected from calcium ion, magnesium ion; and the organic bases capable of providing physiologically acceptable cations are selected from methylamine, dimethylamine, trimethylamine, piperidine, morpholine or tris(2-hydroxyethyl)amine.

All of these salts within the scope of the present invention can be prepared using conventional methods. During the preparation of the compounds of general formula I, solvates and salts thereof, polycrystalline or co-crystal may occur under different crystallization conditions.

### Preparation of compound I

In order to prepare the compounds described in general formula I of the present invention, based on the structure of general formula I, the preparation of general formula I can be obtained by the following synthesis routes. 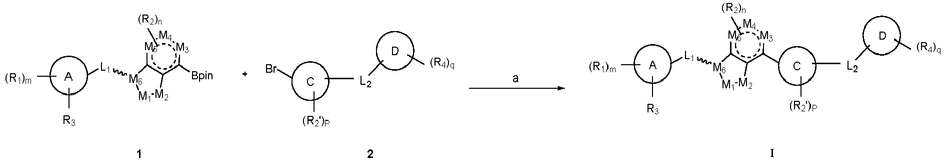
(a) providing the target product I by Suzuki coupling reaction catalyzed by appropriate palladium catalyst with intermediates **1** and **2** as raw materials;
   wherein, the preparation method of intermediate **1** (refer to WO 2018195321, WO2018005374 and WO2019023575) is as follows: 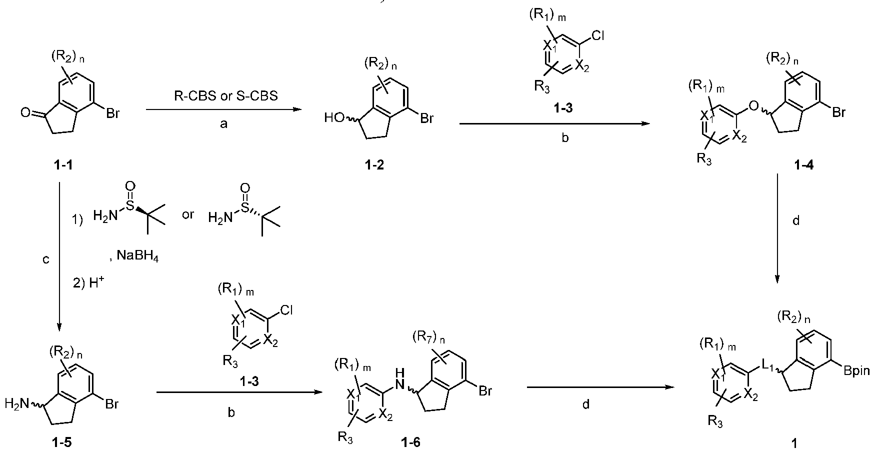
   providing chiral alcohol **1-2** with compound **1-1** as raw material with chiral adjuvant (e.g., R/S-CBS) and reducing agent (e.g., borane);
(b) obtaining the intermediate **1-4** (or **1-6**) by coupling reaction (such as Suzuki, Buchwald, etc.) of **1-2** (or **1-5**) and **1-3** with suitable palladium catalyst and ligand;
(c) providing protected chiral amino compound by reaction of compound **1-1** and R/S-tert-butyl sulfonimide under Lewis acid (such as ethyl tetrasticotitanate) and reducing reagent (such as lithium aluminum hydride, sodium borohydride, etc.), and then removing the protecting group under acidic condition to obtain chiral amino compound **1-5**;
(d) obtaining the intermediate **1** by Suzuki coupling reaction between **1-4** (or **1-6**) and bis(pinacolato)diboron under suitable palladium catalyst and ligand.

The preparation method of intermediate **2** is as follows:
Method 1: 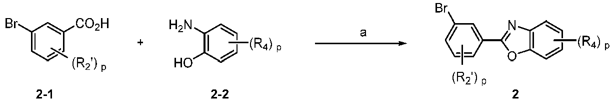 providing intermediate **2** by reacting compounds **2-1** and **2-2** under dehydrating agent (such as concentrated sulfuric acid, PPA, etc.).
Method 2: 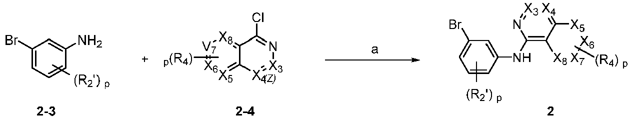 providing intermediate 2 by nucleophilic substitution with compounds 2-3 and 2-4 as raw materials under acidic or alkaline condition.

In addition, the starting materials and intermediates in the above reactions are readily available, and each step of the reactions can be easily synthesized according to the reported literature or by conventional methods in organic synthesis for those skilled in the art. The compound described in general formula I may be present as a solvate or a non-solvate, and different solvates may be obtained by crystallization with different solvents.

### Pharmaceutical composition and administration method

Since the compounds of the present invention have excellent inhibitory activity against PD-1/PD-L1 interaction, the compound of the present invention and various crystal forms thereof, pharmaceutically acceptable inorganic or organic salts, hydrates or solvates thereof, and pharmaceutical composition containing the compound according to the present invention as main active ingredient can be used to prevent and/or treat (stabilize, alleviate or cure) a disease associated with PD-1/PD-L1 interaction (eg, cancer, infectious disease, autoimmune disease).

The pharmaceutical composition of the invention comprises the compound of the present invention in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg compounds of the invention per dose, preferably, 10-200mg compounds of the present invention per dose. Preferably, the "dose" is a capsule or tablet.

"Pharmaceutically acceptable carrier" means one or more compatible solids or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral administration, parenteral (intravenous, intramuscular or subcutaneous) administration.

Solid dosage forms for oral administration include capsules, tablets, pills, powders and granules. In these solid dosage forms, the active compounds are mixed with at least one conventional inert excipient (or carrier), such as sodium citrate or CaHPO4, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or a combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Compounds of the present invention can be administrated alone, or in combination with any other pharmaceutically acceptable compounds (such as other anticaner agents).

In the case of co-administration, the pharmaceutical composition can also include one or more (2, 3, 4, or more) other pharmaceutically acceptable compounds. One or more (2, 3, 4, or more) other pharmaceutically acceptable compounds may be used simultaneously, separately or sequentially with the compound of the present invention for the prevention and/or treatment of PD-1/PD-L1 interation related diseases.

When the pharmaceutical compositions are used, a safe and effective amount of compound of the present invention is applied to a mammal (such as human) in need thereof, wherein the dose of administration is a pharmaceutically effective dose. For a person weighed 60 kg, the daily dose is usually 1-2000 mg, preferably 20-500mg. Of course, the particular dose should also depend on various factors, such as the route of administration, patient healthy status, which are well within the skills of an experienced physician.

### The main advantages of the present invention include:

(1) The compounds of the present invention have high inhibitory activity on PD-1/PD-L1 interaction, strong binding ability to PD-L1 protein, and the ability to relieve the inhibition of IFNγ by PD-L1.
(2) The compounds of the present invention have better solubility; low toxicity to normal cells, so they can be administered to a subject in a larger dosage range.
(3) Compared with the compounds of the prior art, the compounds of the present invention have better solubility, so they have good druggability. Compared with the existing compounds, the compounds of the present invention show good bioavailability in *in vivo* experiments. In addition, compared with existing compounds, the compounds of the present invention can be easily made into pharmaceutically acceptable salts, thus facilitating further formulation.
(4) *In vivo* pharmacodynamic studies show that the compounds of the present invention can significantly inhibit the growth of subcutaneous tumors in terms of tumor volume and weight, and can significantly increase the number of lymphocytes in the blood and spleen of mice.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

The experimental materials and reagents used in the following examples can be commercially available unless otherwise specified.

### General Materials and Test Methods:

The instruments and raw materials involved in the examples are described as follows:
H NMR spectra were obtained by Bruker AV-400 (400MHz) NMR analysis.

Chemical shifts were recorded with tetramethylsilane as an internal standard and were expressed in ppm (CDCl₃: δ 7.26 ppm). The recorded data information was as follows: chemical shifts and their splitting and coupling constants (s: singlet; d: doublet; t: triplet; q: quartet; br: broad; m: multiplet).

Unless otherwise necessary, mass spectral data were analyzed by using LC/MS spectrometer of Finnigan LCQ Advantage, and all reactions were conducted under dry argon protected anhydrous and oxygen-free conditions. The solid metal organic compounds were stored in an argon-protected dry box.

Tetrahydrofuran and ether were obtained by distillation, in which sodium metal and benzophenone were added. Dichloromethane, pentane and hexane were treated with calcium hydride.

The special raw materials and intermediates involved in the present invention are customized and provided by Tianjin Longwood Pharmaceutical Co., Ltd., etc., and all other chemical reagents are purchased from reagent suppliers such as Shanghai Chemical Reagent Company, Aldrich, and Acros, etc.. If the intermediates or products required for the reaction in the synthesis process are not enough for the next step test, the synthesis is repeated several times to sufficient amount.

Unless otherwise specified, the raw materials and reagents involved in the present invention can be commercially available or can be purchased through customized processing.

The compounds of the present invention may contain one or more asymmetric centers, and thus the series of compounds may be in racemic or single enantiomeric form. The compounds prepared in the present invention are heterocyclic compounds with a purity of more than 95%, and the structural characterization of each final product is determined by MS or/and hydrogen spectral nuclear magnetic resonance (¹H NMR) analysis, respectively. The following examples illustrate the synthesis of various compounds and intermediates of the present invention.

### Example 1 Synthesis of compound 1

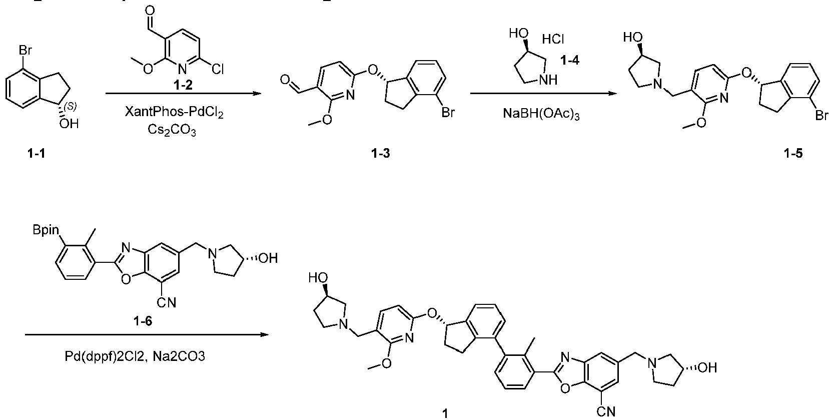

### Step 1-1:

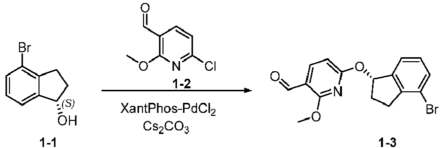

Under the protection of N₂, compound **1-1** (5.0 g, 23.6 mmol, WO2012158550), **1-2** (4.4 g, 25.9 mmol), Xantphos-PdCl₂ (892 mg, 1.18 mmol) and Cs₂CO₃ (15.4 g, 47.2 mmol) were added to dioxane (100 mL) and the mixture was reacted at 95 °C for 2 hours. TLC showed that the reaction was complete. The reaction solution was filtered, and the filtrate was evaporated by rotary evaporation and purified by column chromatography (EA: HEP=1:30) to obtain 4.8 g of pale-yellow solid. MS-APCI: 348 [M+H]⁺.

### Step 1-2:

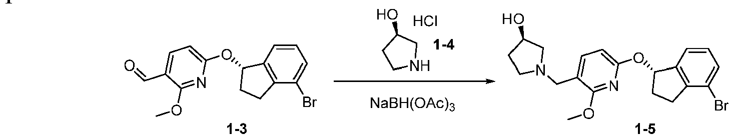

Compound **1-3** (4.8 g, 13.8 mmol), **1-4** (2.5 g, 20.7 mmol) were added to DCM, then 2.8 g of TEA was added. After stirred at room temperature for 1 h, NaBH(OAc)₃ (4.4 g, 20.7 mmol) was added. After 30 minutes, the reaction was completed as shown by TLC. The reaction solution was washed with saturated NaHCOs, extracted with DCM. DCM layer was dried and the reaction solution was evaporated by rotary evaporation. The mixture was purified by a column (MeOH: DCM=1:20) to obtain 5.1 g of product as pale yellow oily viscous substance. MS-APCI: 419 [M+H]⁺.

### Step 1-3:

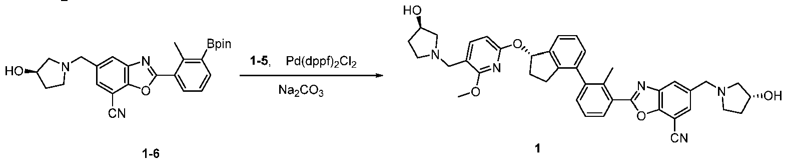

Compounds **1-6** (200 mg, 0.435 mmol, WO2018119266), **1-5** (219 mg, 0.522 mmol), Pd(dppf)₂Cl₂ (36 mg, 0.044 mmol) and Na₂CO₃ (138 mg, 1.3 mmol) were added into the reaction flask. The reaction system was degassed and protected by N₂. 3 mL dioxane/0.6 mL water was injected into the reaction flask and the mixture was reacted at 90 °C for 2 hours. The reaction was completed as shown by TLC test. The reaction solution was washed with water, extracted with EA, dried, and evaporated by rotary evaporation. The mixture was purified by column to obtain 42.6 mg of white solid. MS-APCI: 672 [M+H]⁺.

### Example 2 Synthesis of compound 2

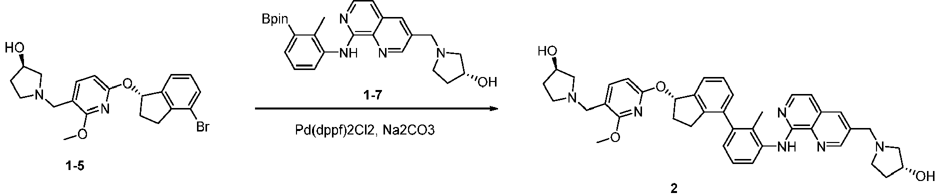

### Step 2-1:

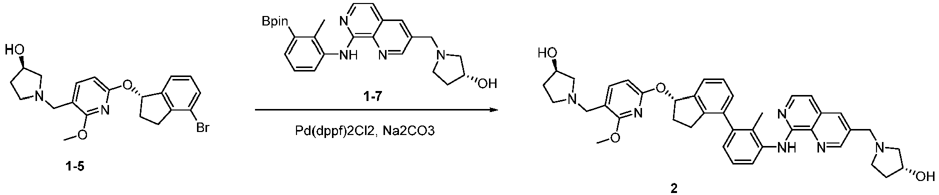

Compounds **1-7** (200 mg, 0.43 mmol, WO 2018119286), **1-5** (218 mg, 0.52 mmol), Pd(dppf)₂Cl₂ (35 mg, 0.043 mmol) and Na₂CO₃ (138 mg, 1.3 mmol) were added into the reaction flask, the reaction system was degassed and protected by N₂. 3 mL dioxane/0.6 mL water was injected into the reaction flask and the mixture was reacted at 90 °C for 2 hours. The reaction was completed as shown by TLC test. The reaction solution was washed with water, extracted with EA, dried, and evaporated by rotary evaporation. The mixture was purified by column to obtain 12.6 mg of pale-yellow solid. MS-APCI: 673 [M+H]⁺.

### Example 3 Synthesis of compound 3

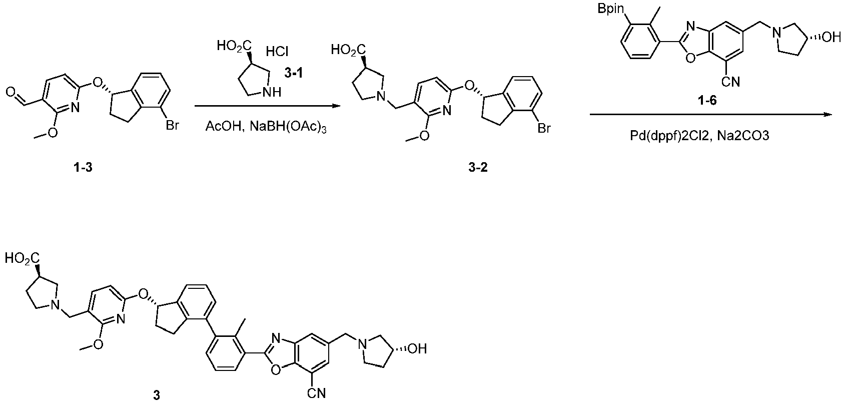

### Step 3-1:

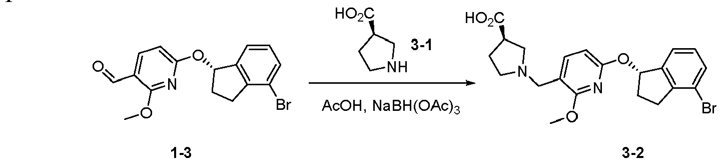

Compound **1-3** (1 g, 2.87 mmol) and **3-1** (661 mg, 5.74 mmol, dissolved in 1.5 mL of AcOH) were added to DCM, then 0.5 g of TEA was added. After stirred at room temperature for 1 h, NaBH(OAc)₃ (1.82 g, 8.61 mmol) was added. After 12 h, the reaction was completed as shown by TLC test. The reaction solution was washed with saturated NaHCO₃, extracted with DCM. DCM layer was dried and the reaction solution was evaporated by rotary evaporation. The mixture was purified by column (MeOH: DCM=1:20) to obtain 0.62 g of product as pale yellow oily viscous substance. MS-APCI: 447 [M+H]⁺.

### Step 3-2:

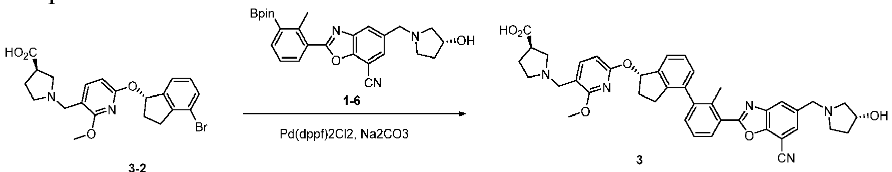

Compounds **3-2** (200 mg, 0.447 mmol), **1-6** (218 mg, 0.52 mmol, WO2018119266), Pd(dppf)₂Cl₂ (37 mg, 0.0447 mmol), and Na₂CO₃ (138 mg, 1.3 mmol) were added into the reaction flask, the reaction system was degassed and protected by N₂. 3 mL dioxane/0.6 mL water was injected into the reaction flask and the mixture was reacted at 90 °C for 2 hours. The reaction was completed as shown by TLC test. The reaction solution was washed with water, extracted with EA, dried and evaporated by rotary evaporation. The mixture was purified by column to obtain 18.5 mg of pale-yellow solid. MS-APCI: 700 [M+H]⁺.

### Example 4 Synthesis of compound 6

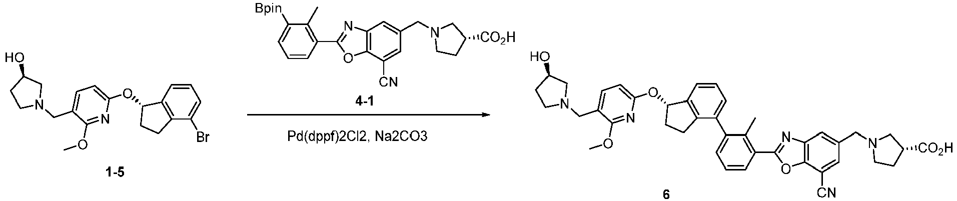

### Step 4-1:

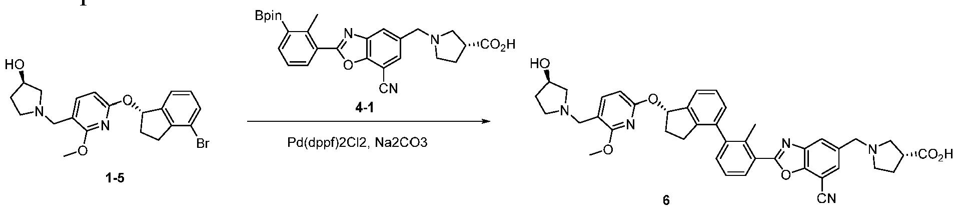

Compounds **1-5** (200 mg, 0.477 mmol), **4-1** (253 mg, 0.52 mmol, WO2018119266), Pd(dppf)₂Cl₂ (39 mg, 0.0477 mmol), and Na₂CO₃ (138 mg, 1.3 mmol) were added into the reaction flask, the reaction system was degassed and protected by N₂. 3 mL dioxane/0.6 mL water was injected into the reaction flask and the mixture was reacted at 90 °C for 2 hours. the reaction was completed as shown by TLC test. The reaction solution was washed with water, extracted with EA, dried and evaporated by rotary evaporation. The mixture was purified by column to obtain 21.5 mg of pale-yellow solid. MS-APCI: 700 [M+H]⁺.

### Example 5 Synthesis of compound 19

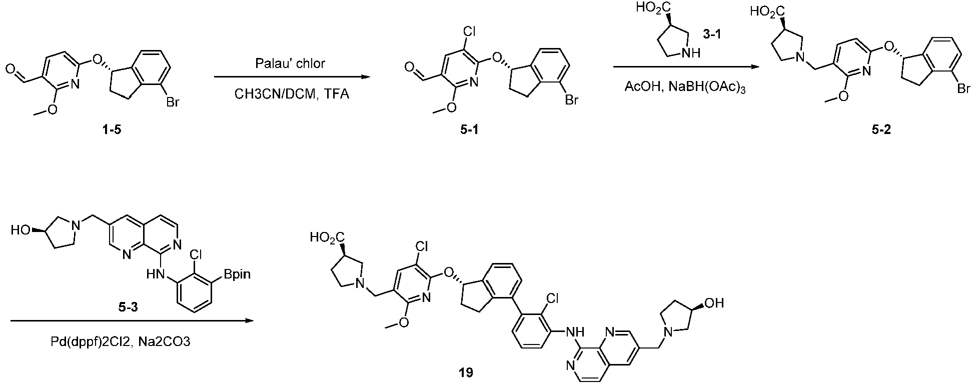

### Step 5-1:

Compound 1-5 (1.5 g, 4.34 mmol) was dissolved in acetonitrile/dichloromethane (20 mL/10 mL), then Palau' Chlor (1 g, 4.77 mmol) and TFA (0.35 mL, 4.77 mmol) were added. The solution was stirred at room temperature overnight and gradually changes from clear to turbid. After the the reaction was completed as shown by LC-MS, the reaction solution was filtered, and the solid was rinsed twice with DCM. The obtained filtrate was washed with saturated salt water and dried over anhydrous sodium sulfate. The mixture was then subjected to a column chromatography to obtain 1.46 g of white product. MS-APCI: 382 [M+H]⁺.

### Step 5-2:

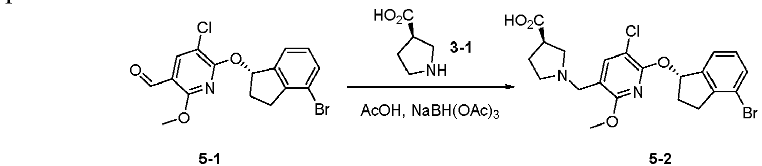

Compounds **5-1** (1 g, 2.61 mmol) and **3-1** (600 mg, 5.22 mmol, dissolved in 1.5 mL of AcOH) were added to DCM, then 0.5 g of TEA was added. After stirred at room temperature for 1 h, NaBH(OAc)₃ (1.66 g, 7.83 mmol) was added. After 12 h, the reaction was completed as shown by TLC test. The reaction solution was washed with saturated NaHCOs, extracted with DCM. DCM layer was dried and the reaction solution was evaporated by rotary evaporation. The mixture was purified by column (MeOH:DCM=1:20) to obtain 0.53 g of product as pale yellow oily viscous substance. MS-APCI: 447 [M+H]⁺.

### Step 5-3:

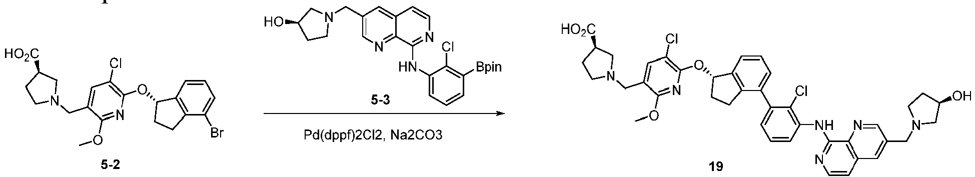

Compounds **5-2** (230 mg, 0.477 mmol), **5-3** (275 mg, 0.57 mmol, WO2018119286), Pd(dppf)₂Cl₂ (39 mg, 0.0477 mmol) and Na₂CO₃ (138 mg, 1.3 mmol) were added into the reaction flask. the reaction system was degassed and protected by N₂. 3 mL dioxane/0.6 mL water was injected into the reaction flask and the mixture was reacted at 90 °C for 2 hours. the reaction was completed as shown by TLC test. TFA was added to the reaction solution to neutral. The mixture was purified by reversed-phase column to obtain 25.4 mg of pale-yellow solid. MS-APCI: 700 [M+H]⁺.

### Example 6 Synthesis of compound 27

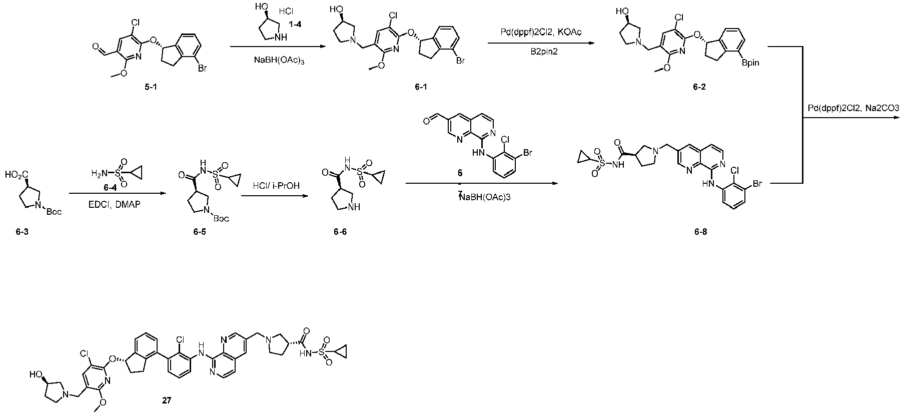

### Step 6-1:

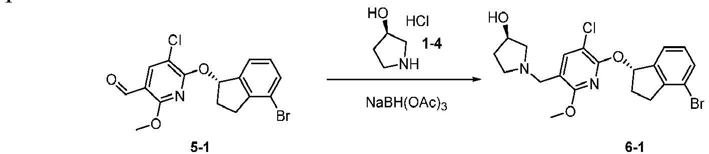

Compound **5-1** (1 g, 2.61 mmol) and **1-4** (645 mg, 5.22 mmol) were added to DCM, then 0.5 g of TEA was added. After stirred at room temperature for 1 h, NaBH(OAc)₃ (1.66 g, 7.83 mmol) was added. After 12 h, the reaction was completed as shown by TLC test. The reaction solution was washed with saturated NaHCOs, extracted with DCM. DCM was dried and the reaction solution was evaporated by rotary evaporation. The mixture was purified by a column (MeOH: DCM=1:20) to obtain 0.6 g of product as pale yellow oily viscous substance. MS-APCI: 453 [M+H]⁺.

### Step 6-2:

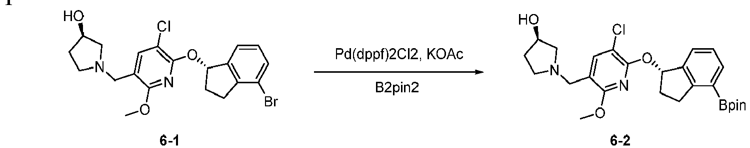

Compound **6-1** (200 mg, 0.44 mmol), B2pin2 (135 mg, 0.53 mmol), Pd(dppf)₂Cl₂ (36 mg, 0.044 mmol) and KOAc (129 mg, 1.3 mmol) were added into the reaction flask. The reaction system was degassed and protected by N₂. 3 mL dioxane was injected into the reaction flask and the mixture was reacted at 90 °C for 2 hours. The reaction was completed as shown by TLC test. The reaction solution was washed with water, extracted with EA, dried and evaporated by rotary evaporation. The mixture was purified by a column to obtain 180 mg of pale-yellow solid. MS-APCI: 501 [M+H]⁺.

### Step 6-3:

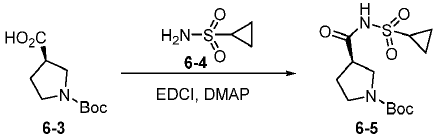

Compounds **6-3** (500 mg, 2.32 mmol), **6-4** (310 mg, 2.56 mmol), DMAP (567 mg, 4.64 mmol) and EDCI (889 mmol, 4.64 mmol) were added into DCM (15 mL) successively and the mixture was stirred at room temperature for 1 h. TLC was used to test the reaction. After the reaction was completed, the reaction solution was washed with 0.5M of HCl solution (5mL×3). Then the organic layer was dried with anhydrous sodium sulfate. The mixture was then subjected to a column chromatography to obtain 548 mg of white solid. MS-APCI: 317 [M+H]⁺.

### Step 6-4:

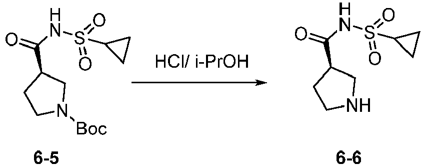

Compound **6-5** (548 mg, 1.72 mmol) was dissolved in isopropanol solution of HCl (12M, 3 mL) and stirred for 30 min at room temperature. The solution was evaporated by rotary evaporation. The mixture was azeotroped with toluene (10 mL) for 3 times and then dried in vacuum to obtain 375 mg of white solid.

### Step 6-5

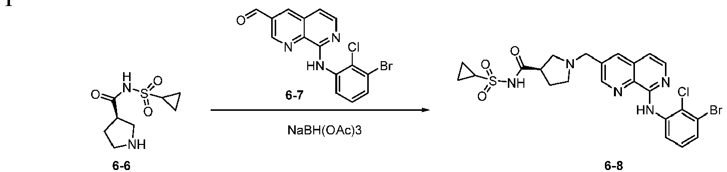

Compounds **6-6** (300 g, 1.37 mmol) and **6-7** (497 mg, 1.37 mmol, WO2018119266) were added to DCM, then 0.5 g of TEA was added. After stirred at room temperature for 1 h, NaBH(OAc)₃ (871 mg, 4.11 mmol) was added. After 12 h, the reaction was completed as shown by TLC test. The reaction solution was washed with saturated NaHCO₃, extracted with DCM. DCM layer was dried and the reaction mixture was evaporated by rotary evaporation. The mixture was purified by column (MeOH: DCM=1:20) to obtain 0.41 g of product as pale yellow oily viscous substance. MS-APCI: 564 [M+H]⁺.

### Step 6-6:

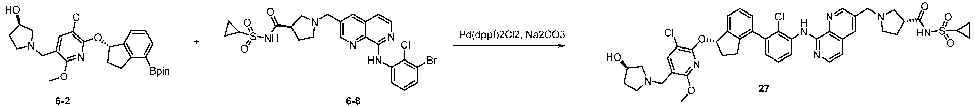

Compounds **6-2** (150 mg, 0.3 mmol), **5-3** (169 mg, 0.3 mmol), Pd(dppf)₂Cl₂ (24 mg, 0.03 mmol) and Na₂CO₃ (95 mg, 0.9 mmol) were added into the reaction flask. The reaction system was degassed and protected by N₂. 3 mL dioxane/0.6 mL water was injected into the reaction flask and the mixture was reacted at 90 °C for 2 hours. The reaction was completed as shown by TLC test. The reaction solution was washed with water, extracted with EA. The mixture was prepared by reversed-phase column to obtain 11.8 mg of pale-yellow solid. MS-APCI: 858 [M+H]⁺.

### Example 7 Synthesis of compound 8

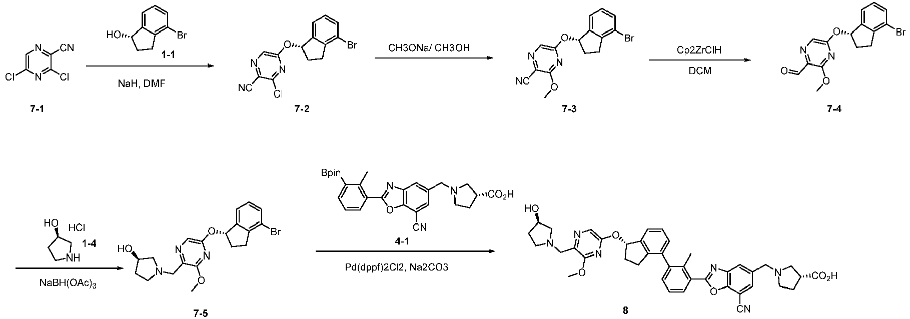

### Step 7-1:

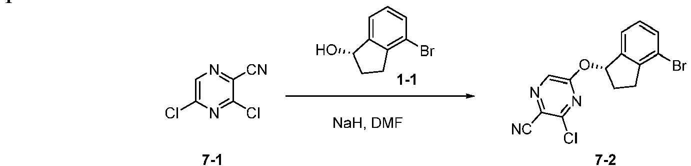

Compound **1-1** (1 g, 4.69 mmol) was dissolved in DMF (10 mL), the mixture was stirred in an ice water bath, then NaH (60%, 135 mg, 5.628 mmol) was added thereto. After stirred for 1 hour, 7-1 (816 mg, 4.69 mmol) was added to the reaction solution, the reaction solution was gradually heated to room temperature and stirred overnight. The reaction was completed as shown by TLC test. The reaction solution was quenched with water. The mixture was diluted to 60 mL with ethyl acetate, washed with saturated salt water, then dried with anhydrous sodium sulfate. The mixture was purified by column chromatography to obtain 1.2 g of solid. MS-APCI: 350 [M+H]⁺.

### Step 7-2:

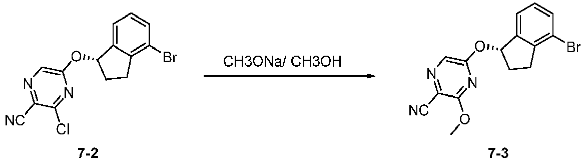

Compound **7-2** (1 g, 2.85 mmol) was dissolved in CH₃OH (5 mL), the methanol solution of CHsONa (5 M, 0.7 mL, 0.34 mmol) was then added thereto, and the solution was stirred overnight at room temperature. The reaction was completed as shown by TLC test. The reaction solution was quenched with water. The mixture was diluted to 60 mL with ethyl acetate, washed with saturated brine, then dried with anhydrous sodium sulfate. The mixture was purified by column chromatography to obtain 0.95 g of solid. MS-APCI: 346 [M+H]⁺.

### Step 7-3:

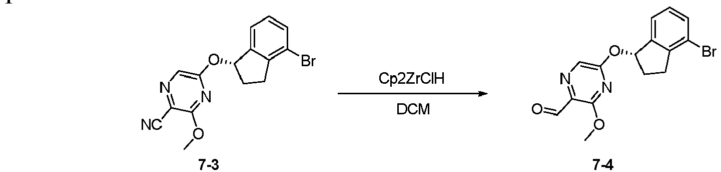

Compound **7-3** (0.9 g, 2.6 mmol) was dissloved in DCM (10 mL), the mixture was stirred in an ice water bath, then Cp₂ZrClH (1.17 g, 4.55 mmol) was added thereto. The mixture was stirred for 1 hour under below 0 °C. The reaction was completed as shown by TLC test. Water (10 mL) was added and the reaction mixture was stirred for 20 minutes and filtered. The solid was washed with DCM, and the combined organic layers was washed with brine, dried with anhydrous sodium sulfate, and purified by column chromatography to obtain 0.5g of solid. MS-APCI: 349 [M+H]⁺.

### Step 7-4:

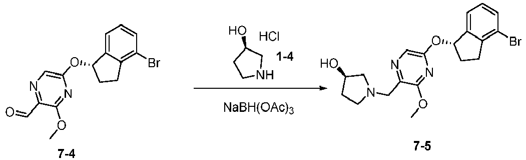

Compounds **7-4** (0.5 g, 1.43 mmol) and **1-4** (354 mg, 2.86 mmol) were added to DCM, then 0.5 g of TEA was added. After stirred at room temperature for 1 h, NaBH(OAc)₃(909 mg, 4.29 mmol) was added. Two hours later, the reaction was completed as shown by TLC test. The reaction solution was washed with saturated NaHCO₃, extracted with DCM. DCM layer was dried, and the mixture was evaporated by rotary evaporation, and purified by column (MeOH: DCM=1:20) to obtain 0.6 g of product as a light yellow oily viscous substance. MS-APCI: 420 [M+H]⁺.

### Step 7-5:

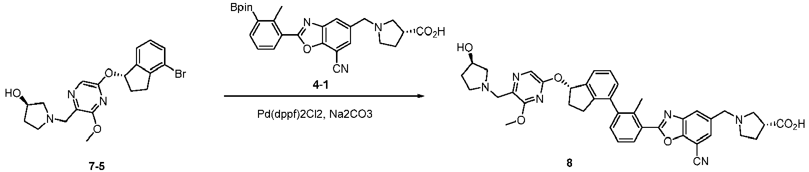

Compounds **7-5** (200 mg, 0.476 mmol), **4-1** (253 mg, 0.52 mmol, WO2018119266), Pd(dppf)₂Cl₂ (39 mg, 0.0477 mmol), and Na₂CO₃ (138 mg, 1.3 mmol) were added into the reaction flask. The reaction system was degassed and protected by N₂. 3 mL dioxane/0.6 mL water was injected into the reaction flask, and the mixture was reacted at 90 °C for 2 hours. The reaction was completed as shown by TLC test. The reaction solution was washed with water, extracted with EA, dried, evaporated by rotary evaporation, and subjected to a column to obtain 23 mg of light yellow solid. MS-APCI: 701 [M+H]⁺.

According to the above synthesis methods, the following compounds were prepared from the corresponding raw materials, and the mass spectral datas are shown in Table 1:

**Table 1**

| Compound | Note | Compound | Note |
|---|---|---|---|
| 4 | 702 [M⁺H]⁺. | 5 | 702 [M⁺H]⁺. |
| 7 | 701 [M⁺H]⁺. | 9 | 701 [M⁺H]⁺. |
| 10 | 721 [M⁺H]⁺. | 11 | 721 [M⁺H]⁺. |
| 12 | 722 [M⁺H]⁺. | 13 | 722 [M⁺H]⁺. |
| 18 | 755 [M⁺H]⁺. | 20 | 754 [M⁺H]⁺. |
| 21 | 754 [M⁺H]⁺. | 22 | 753 [M⁺H]⁺. |
| 23 | 765 [M⁺H]⁺. | 24 | 753 [M⁺H]⁺. |
| 25 | 765 [M⁺H]⁺. | 26 | 857 [M⁺H]⁺. |
| 28 | 871 [M⁺H]⁺. | 29 | 872 [M⁺H]⁺. |
| 30 | 824 [M⁺H]⁺. | 31 | 825 [M⁺H]⁺. |
| 32 | 892 [M⁺H]⁺. | 33 | 893 [M⁺H]⁺. |
| 34 | 788 [M⁺H]⁺. | 35 | 800 [M⁺H]⁺. |
| 36 | 906 [M⁺H]⁺. | 37 | 907 [M⁺H]⁺. |
| 38 | 789 [M⁺H]⁺. | 39 | 801 [M⁺H]⁺. |
| 57 | 739 [M⁺H]⁺. | 58 | 739 [M⁺H]⁺. |
| 59 | 738 [M⁺H]⁺. | 60 | 738 [M⁺H]⁺. |
| 71 | 876 [M⁺H]⁺. | 72 | 877 [M⁺H]⁺. |
| 73 | 772 [M⁺H]⁺. | 74 | 784 [M⁺H]⁺. |
| 82 | 905 [M⁺H]⁺. | 83 | 885 [M⁺H]⁺. |
| 87 | 868 [M⁺H]⁺. | 88 | 883 [M⁺H]⁺. |
| 91 | 780 [M⁺H]⁺. | 92 | 764 [M⁺H]⁺. |
| 95 | 781 [M⁺H]⁺. | 96 | 788 [M⁺H]⁺. |
| 97 | 768 [M⁺H]⁺. | 98 | 772 [M⁺H]⁺. |
| 105 | 748 [M⁺H]⁺. | 106 | 741 [M⁺H]⁺. |

### Biological Test

### Example A: PD-1/PD-L1 Homogeneous Time-Resolved Fluorescence (HTRF) Binding Assay

Assays were performed in standard black 384-well polystyrene plates and a final volume was 20 µL. The inhibitor was first serially diluted with DMSO and added to the wells of the plate, then other reaction components were added. The final concentration of DMSO in the assay was 1%. Assays were performed at 25°C in PBS buffer (pH 7.4) containing 0.05% Tween-20 and 0.1% BSA. Recombinant human PD-L1 protein (19-238) with a His-tagged at the C-terminus was purchased from AcroBiosystems (PD1-H5229). Recombinant human PD-1 protein (25-167) with an Fc tag at the C-terminus was also purchased from AcroBiosystems (PD1-H5257). The PD-L1 and PD-1 proteins were diluted in assay buffer and then 0.1 µl of the solution was extracted and added to the wells of the plate. Plates were centrifuged and proteins and inhibitors were preincubated for 40 minutes. After incubation, 0.1 µl of HTRF detection buffer containing europium blocking labeled anti-human IgG (PerkinElmer-AD0212), Fc-specific and anti-His SureLight^{®}-Allophycocyanin (APC, PerkinElmer-AD0059H) conjugated antibodies was added. After centrifugation, the plates were incubated at 25°C for 60 minutes. Data was read in a PHERAstar FS plate reader (665nm/620nm ratio). Final concentrations in the assay were ~3 nM of PD1, 10 nM of PD-L1, 1 nM of europium anti-human IgG, and 20 nM of anti-His-allophycocyanin. Activity data were fitted using GraphPad Prism 5.0 software to derive IC50 values for inhibitors. The IC50 values of the compounds exemplified in the examples are expressed in the following manner: IC50: + = ≤100 nM; ++ =100 nM-1000 nM; +++ = >1000 nM. The data of the example compounds obtained by PD-1/PD-L1 homogeneous time-resolved fluorescence (HTRF) binding assay described in Example A were provided in Table 1.

**Table 1.**

| Compound number | PD-1/PD-L1 HTRF IC50(nM) |
|---|---|
| 3 | + |
| 4 | + |
| 5 | + |
| 6 | + |
| 7 | + |
| 8 | + |
| 9 | + |
| 10 | + |
| 12 | + |
| 13 | + |

All documents mentioned herein are incorporated by reference in the present invention as if each document was individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A compound shown in Formula I below, or optical isomers, cis-trans isomers, hydrates, solvates thereof, or pharmaceutically acceptable salts thereof: 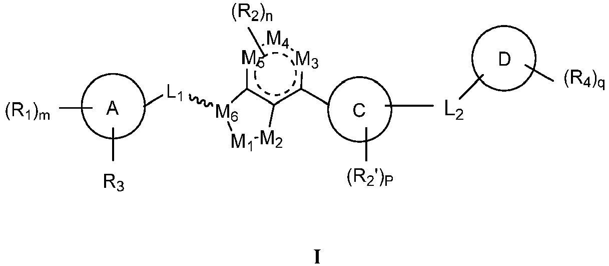
wherein, n, m, p and q are each independently selected from 0, 1, 2, 3 or 4;
L₁ and L₂ are each independently selected from the group consisting of chemical bond, substituted or unsubstituted C₁-C₄ alkylene, substituted or unsubstituted C₂-C₄ alkenylene, substituted or unsubstituted C₂-C4 alkenyl, -S-, -O-, substituted or unsubstituted -NH-, -S(O)-, -S(O)₂-, substituted or unsubstituted -NHC(O)NH-, 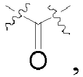 substituted or unsubstituted 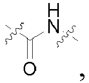 substituted or unsubstituted 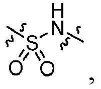 substituted or unsubstituted 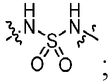
M₁ and M₂ are each independently selected from the group consisting of C(R₆)₂, NR₆, O, S, SO, SO₂; wherein, R₆ is selected from the group consisting of H, chlorine, bromine, fluorine, iodine, cyano, hydroxyl, nitro, NR_{f}, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, -C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, substituted or unsubstituted C₂-C₆ alkenyl, substituted or unsubstituted C₂-C₆ alkynyl, -C(=O)-substituted or unsubstituted C₂-C₆ alkenyl, and -C(=O)-substituted or unsubstituted C₂-C₆ alkynyl;
M₃, M₄ and M₅ are each independently selected from the group consisting of chemical bond, CR₆, N, NR₆, O, S, SO and SO₂;
M₆ is selected from the group consisting of CR₆, N;
and the 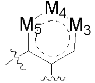 is aromatic ring; 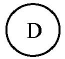
is a group having the following structure: 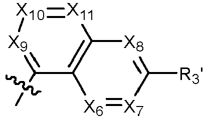 or 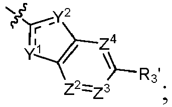
wherein,
X⁶, X⁷, X⁸, X⁹, X¹⁰, X¹¹, Z², Z³, and Z⁴ are each independently selected from the group consisting of N, N-O, CRₐ; wherein, the Rₐ is selected from the group consisting of H, chlorine, bromine, fluorine, iodine, cyano, hydroxyl, nitro, NR_{f}, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, -C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, substituted or unsubstituted C₂-C₆ alkenyl, substituted or unsubstituted C₂-C₆ alkynyl, -C(=O)-substituted or unsubstituted C₂-C₆ alkenyl, and -C(=O)-substituted or unsubstituted C₂-C₆ alkynyl;
Y¹ and Y² are each independently selected from the group consisting of CH, CH₂, NH, N, N-O, CF, CRₐ, O, S, SO or SO₂;
-̅ -̅ -̅ is single bond or double bond;
and 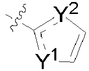 is an aromatic or non-aromatic fragment; 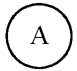
is selected from the group consisting of substituted or unsubstituted 5-12membered heteroaryl, substituted or unsubstituted C₆-C₁₀ aryl, substituted or unsubstituted 5-12 membered heterocyclyl, substituted or unsubstituted 5-12 membered C₅-C₁₂ ring group, wherein the 5-12-membered heteroaryl and 5-12 membered heterocyclyl have 1-4 heteroatoms selected from B, P, N, O, S, wherein P, N, O as ring atoms can be oxygenated and one or more cyclic carbon atoms can be replaced with carbonyl; 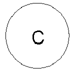
is a divalent group formed by a ring selected from the group consisting of 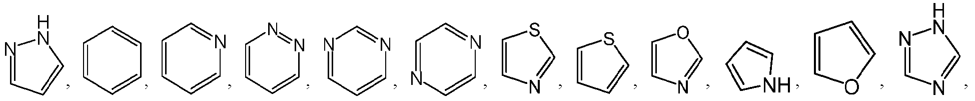 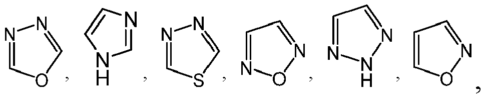 wherein bonding position of the ring can be N or C;
R₁, R₂, R₂' and R₄ are each independently selected from the group consisting of H, halogen, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted C₃-C₈ cycloalkyl, oxy (i.e.=O), =NR_{f}, -CN, hydroxyl, NR_{d}Rₑ (e.g. amino), substituted or unsubstituted C₁-C₆ amino, substituted or unsubstituted -(C₁-C₆ alkylene)-NH-(C₁-C₆ alkylene), carboxyl, substituted or unsubstituted C₆-C₁₀ aryl, substituted or unsubstituted 5-12 membered heteroaryl with 1-3 heteroatoms substituted or unsubstituted 5-12 membered heterocyclyl with 1-4 heteroatoms, substituted or unsubstituted 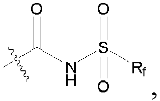 substituted or unsubstituted 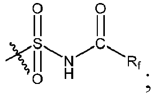 or -(L₁ₐ)ᵣ-(L₂ₐ)ₛ-(L₃ₐ)ₛ-;
R₃ and R₃'are 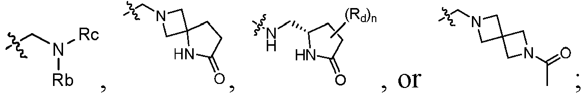 wherein R_{b}, R_{c} and R_{d} are each independently selected from the group consisting of H, substituted or unsubstituted C₁-C₈ alkyl; or R_{b} and R_{c} together with the adjacent N atom form substituted or unsubstituted 5-10 membered heterocyclyl with 1-3 heteroatoms selected from N, S and O;
each L₁ₐ is each independently selected from the group consisting of chemical bond, substituted or unsubstituted C₁-C₇ alkylene, substituted or unsubstituted C₂-C₄ alkenylene, substituted or unsubstituted C₂-C₄ alkynylene, -S-, -O-, substituted or unsubstituted -NH-, -S(O)-, -S(O)₂-;
L₂ₐ is selected from the group consisting of substituted or unsubstituted C₆-C₁₂ arylene, substituted or unsubstituted 5-12-membered heteroarylene with 1-3 heteroatoms, substituted or unsubstituted C₃-C₈ cycloalkylene, substituted or unsubstituted 5-10 membered heterocyclylene with 1-3 heteroatoms;
L₃ₐ is selected from the group consisting of H, substituted or unsubstituted C₁-C₁₀ alkyl, C₁-C₁₀ aryl, -CN, hydroxyl, amino, carboxyl, -CO-NH-SO₂-R_{g}, -NH-SO₂-R_{g}, -SO₂-NH-CO-R_{g};
R_{d}, Rₑ and R_{g} are each independently selected from the group consisting of H, substituted or unsubstituted C₁-C₆ alkyl, substituted or unsubstituted C₃-C₁₀ cycloalkyl, substituted or unsubstituted C₆-C₁₀ aryl;
R_{f} is selected from the group consisting of H, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ heteroaryl, cyano, -C(=O)-NR_{d}Rₑ, - C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, -C(=O)-substituted or unsubstituted C₂-C₆ alkynyl; unless otherwise specified, the "substituted" means being substituted by one or more (such as 2, 3, 4, etc.) substituents selected from the group consisting of halogen (including but not limited to -F, Cl, Br), -CH₂Cl, -CHCl₂, -CCl₃, -CH₂F, - CHF₂, -CF₃, oxo, -CN, hydroxyl, amino, C₁-C₆ alkyl amino, carboxyl, -NHAc, or substituted or unsubstituted groups which are selected from C₁-C₆ alkyl, C₁-C₆ alkoxy, C₆-C₁₀ aryl, C₃-C₈ cycloalkyl, halogenated C₆-C₁₀ aryl, 5-10 membered heteroaryl with 1-3 heteroatoms selected from N, S and O, and 5-10 membered heterocyclyl with 1-3 heteroatoms selected from N, S and O; the substituent is selected from the group consisting of halogen, hydroxyl, carboxyl, cyano, C₁-C₆ alkoxy, C₁-C₆ alkylamino;
among the above formulas, any one of the heteroatoms is selected from the group consisting of B, P, N, S and O.

2. The compound of claim 1, or optical isomers, hydrates, solvates thereof, or pharmaceutically acceptable salts thereof, wherein ring 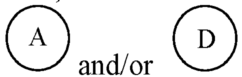 have substituents as shown in Formula IV below: 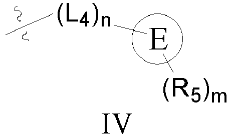
wherein, each L₄ is independently selected from the group consisting of substituted or unsubstituted C₁-C₄ alkylene, -S-, -O-, -NRₐ-, -S(O)-, -S(O)₂-; preferably substituted or unsubstituted C₁-C₄ alkylene, provided that the structure formed by each L₄ is chemically stable; 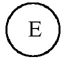
is selected from the group consisting of substituted or unsubstituted C₅-C₁₀ cycloalkyl, substituted or unsubstituted 3-10 membered heterocyclyl with 1-3 heteroatoms selected from B, P, N, S and O; preferably, 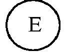 is nitrogen-containing 3-8 membered heterocyclyl;
each R₅ is each independently selected from the group consisting of substituted or unsubstituted C₁-C₆ alkyl, -CN, hydroxyl, amino and carboxyl; wherein, the substituent is selected from the group consisting of halogen, hydroxyl, carboxyl, cyano, C₁-C₆ alkoxy.

3. The compound of claim 1, or isomers, optical isomers, hydrates, solvates thereof, or pharmaceutically acceptable salts thereof, wherein 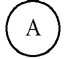 is the structure shown in the following formula: 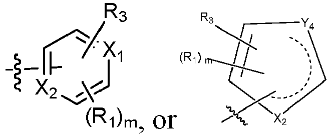 wherein, X₁, X₂ and Y₄ are each independently selected from CH (the CH can be substituted by R₁ or R₃), N, O, S and NH (the NH can be substituted by R₁ or R₃).

4. The compound of claim 1, or optical isomers, cis-trans isomers, hydrates, solvates thereof, or pharmaceutically acceptable salts thereof, wherein the compound has the structure shown in Formula I-a or I-b as follows: 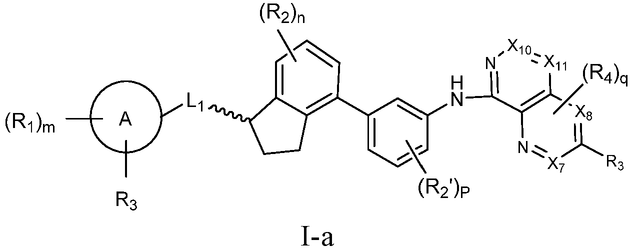 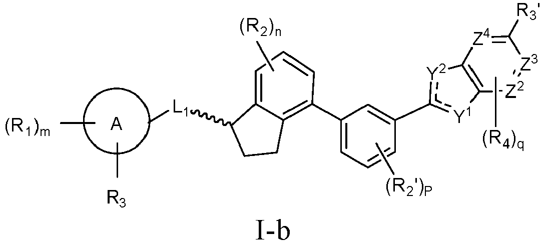 wherein,
R₃ and R₃' are each independently 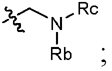 wherein, R_{b} and R_{c} together with adjacent N atom form substituted or unsubstituted 5-10 membered heterocyclyl (preferably 5-7 membered heterocyclyl) with 1-3 heteroatoms selected from N, S and O;
other groups are defined as described above.

5. The compound of claim 3, or optical isomers, cis-trans isomers, hydrates, solvates thereof, or pharmaceutically acceptable salts thereof, wherein in the compound, R₃ and R₃' are each independently

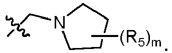

6. The compound of claim 1, or optical isomers, cis-trans isomers, hydrates, solvates thereof, or pharmaceutically acceptable salts thereof, wherein the compound is selected from the group consisting of:

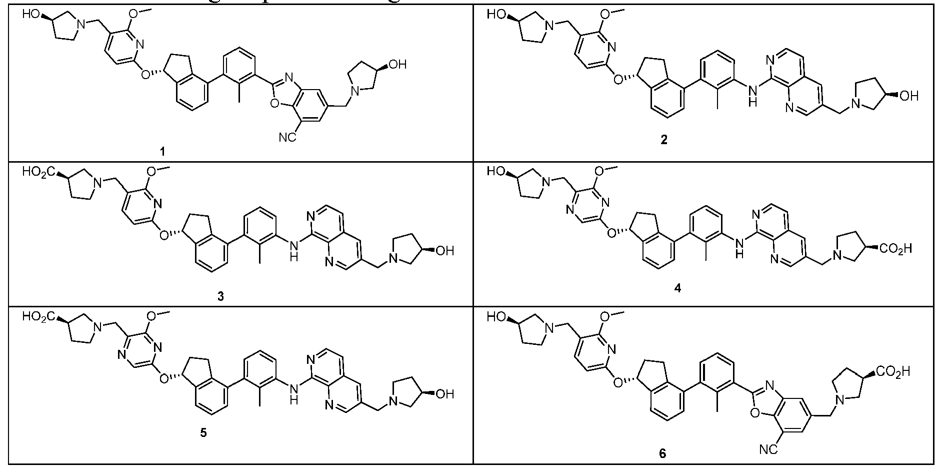

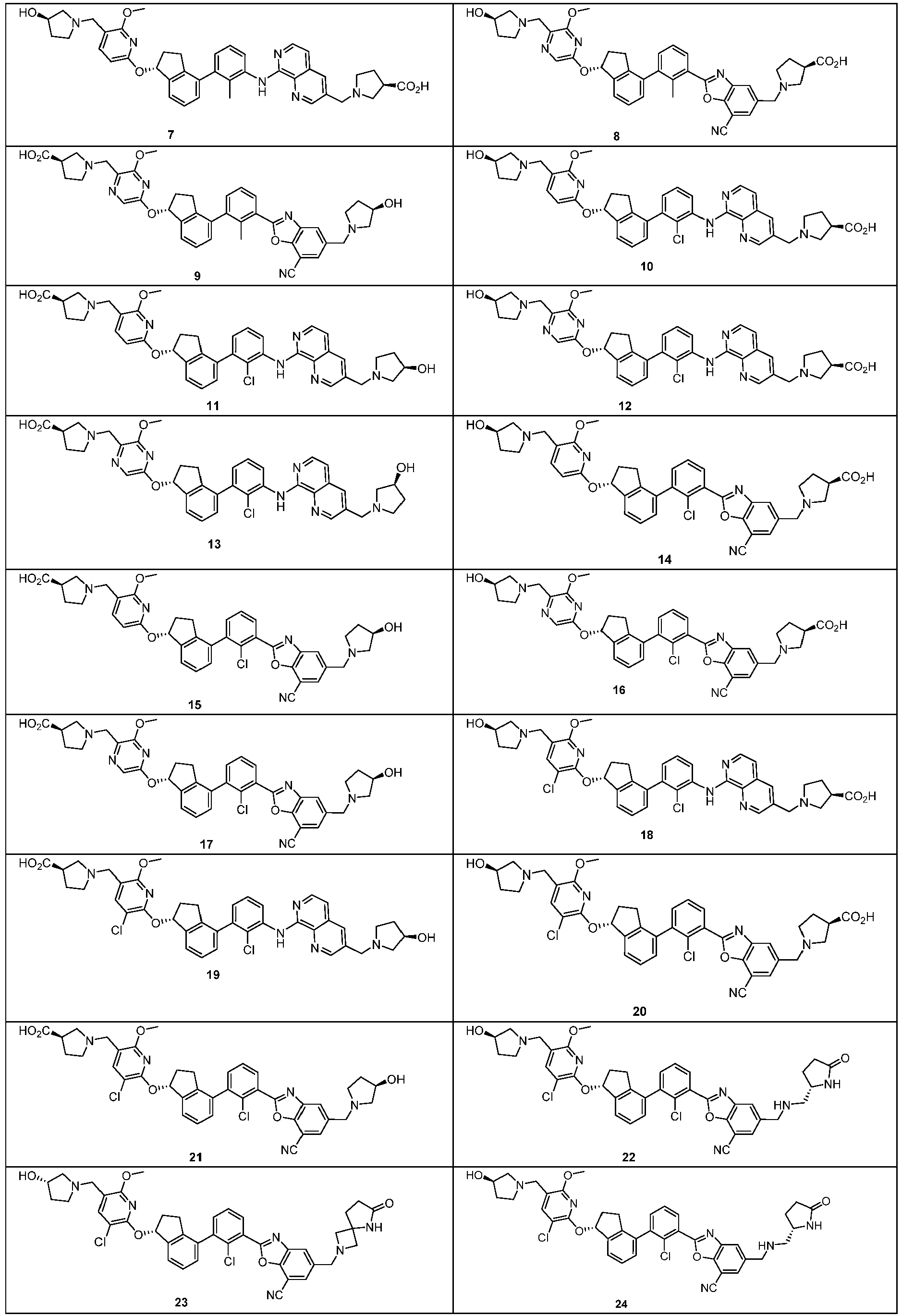

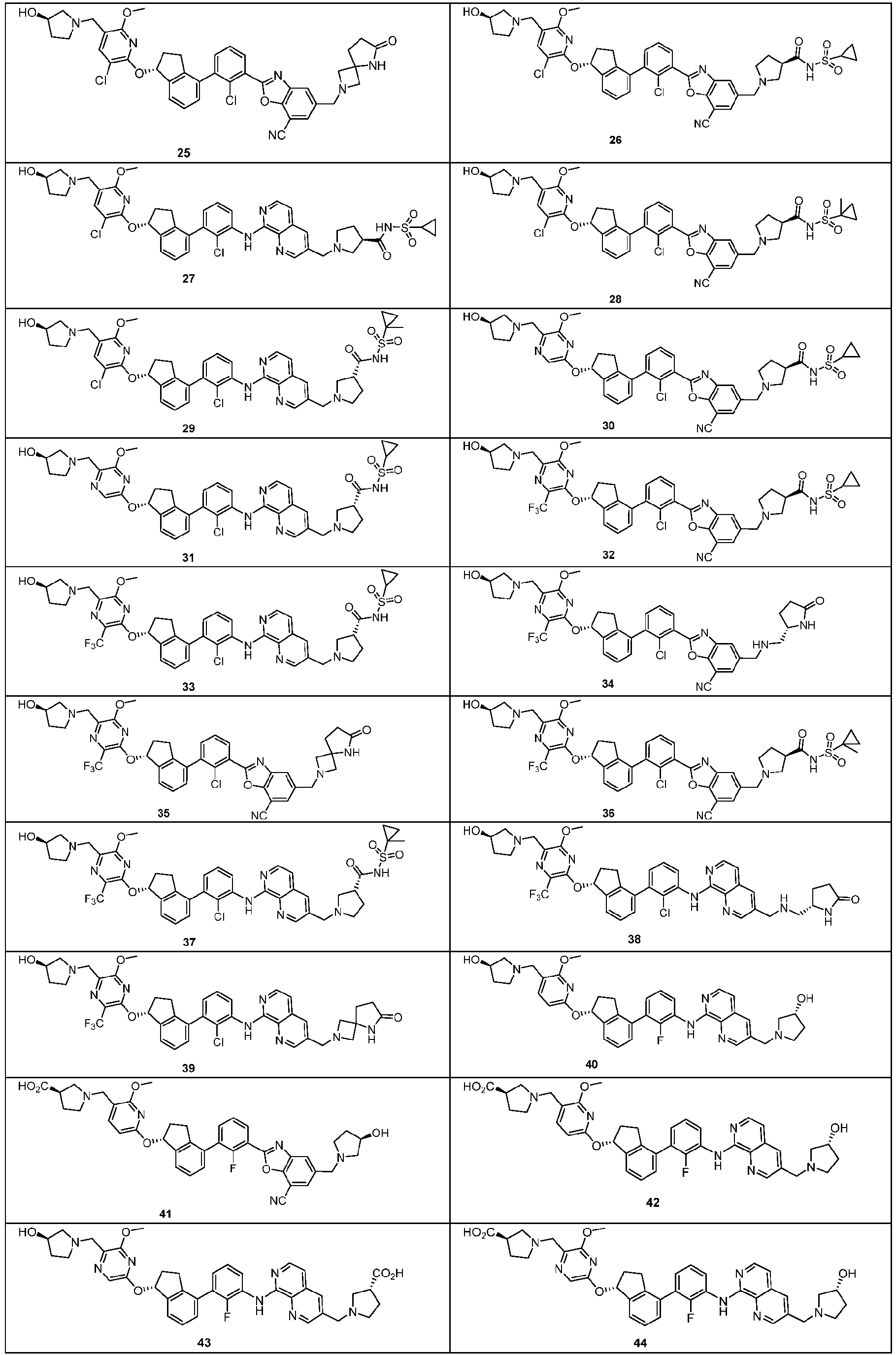

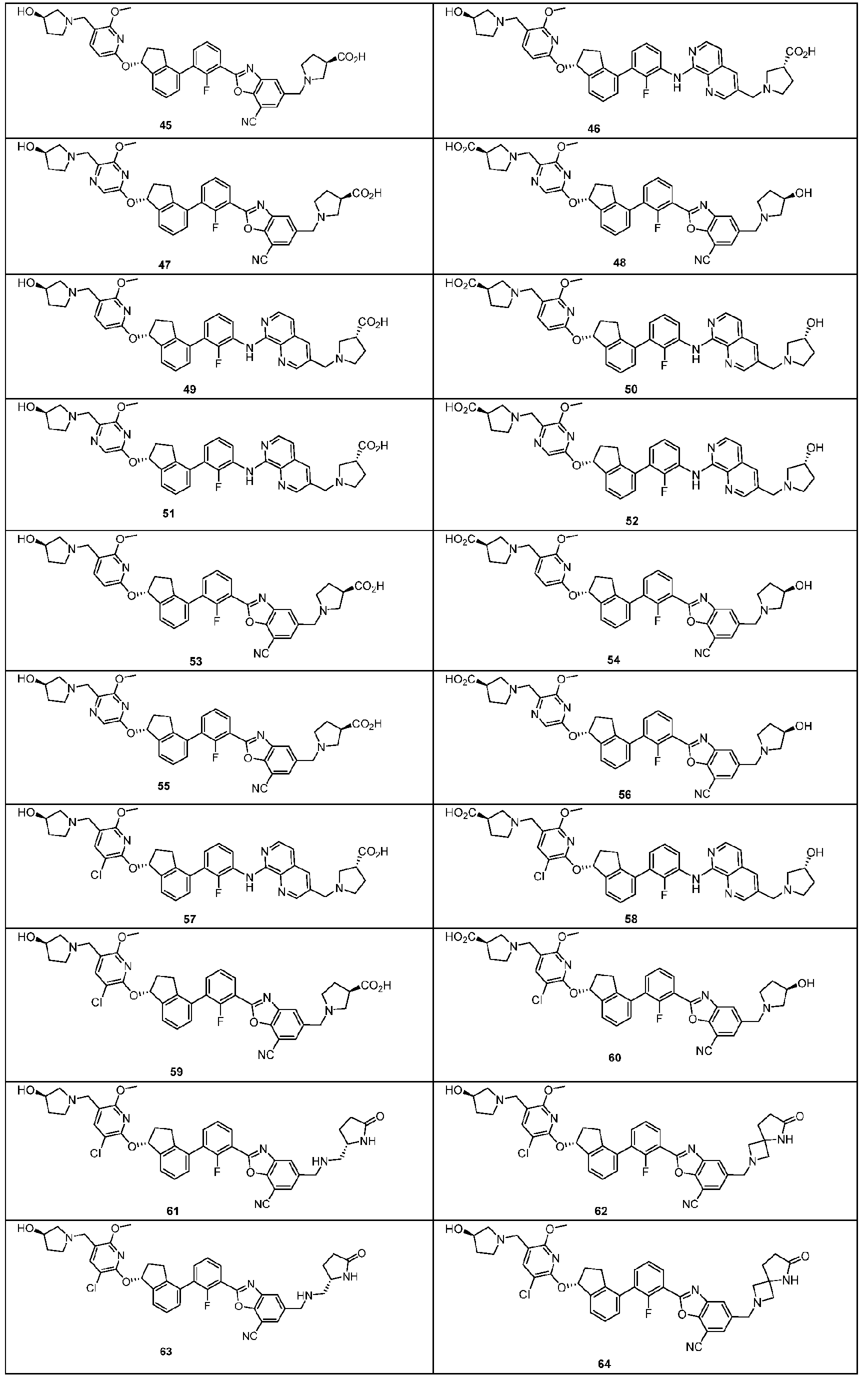

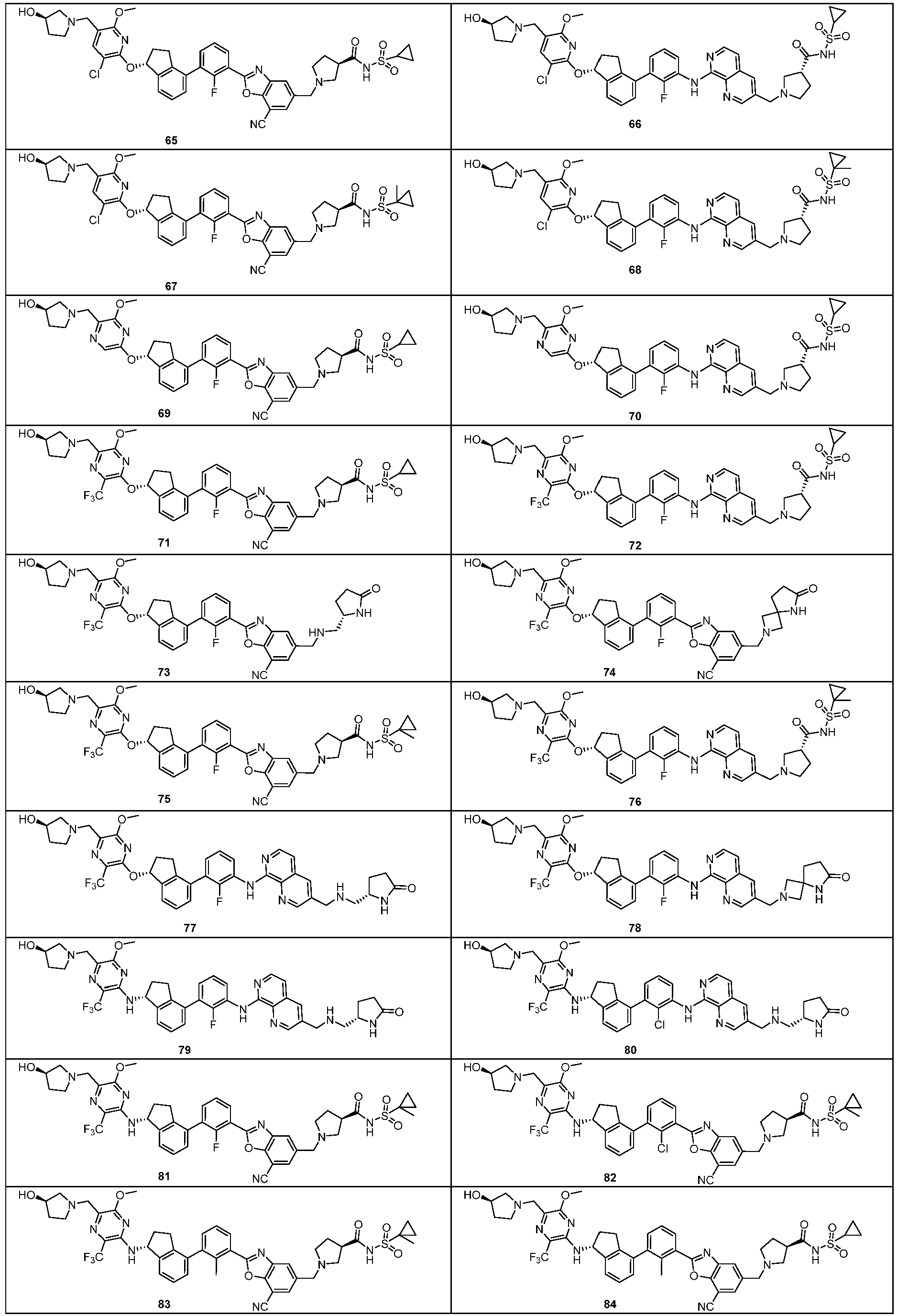

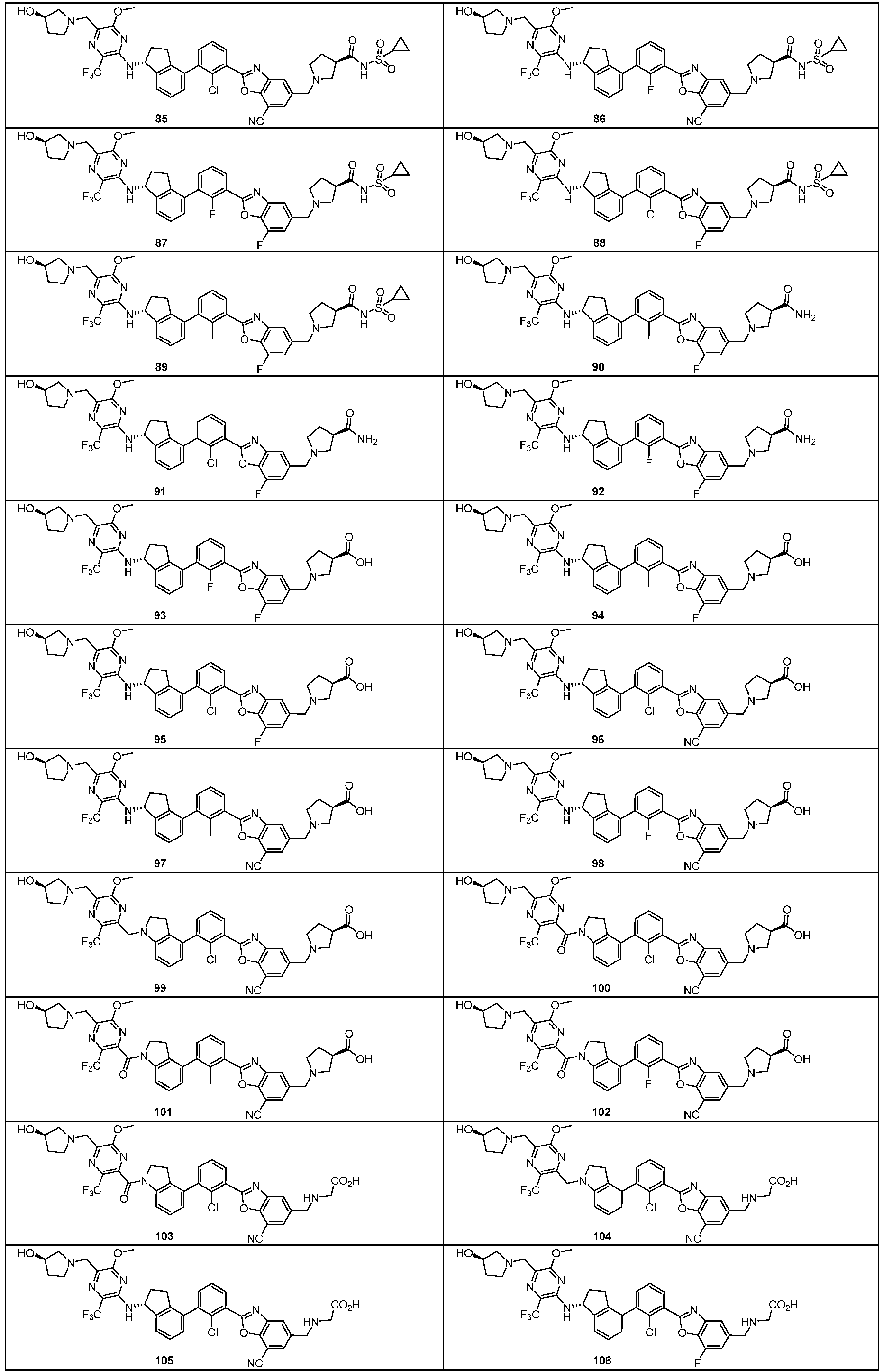

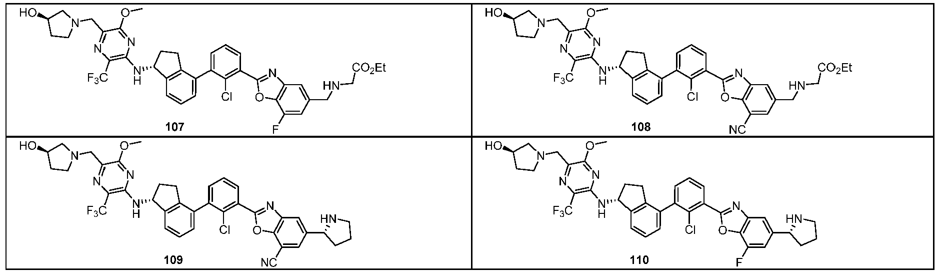

7. A method for preparing compound of claim 1, wherein the method comprises the following steps: 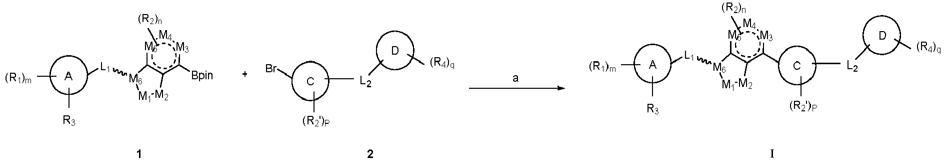
(a) providing the target product I by Suzuki coupling reaction catalyzed by appropriate palladium catalyst with intermediates **1** and **2** as raw materials;
wherein, the definition of each group is as described in claim 1.

8. A pharmaceutical composition, comprises (1) the compound of claim 1 or stereoisomers or tautomers thereof, or pharmaceutically acceptable salts, hydrates or solvates thereof; (2) pharmaceutically acceptable carriers.

9. A use of the compound of claim 1 or stereoisomers or tautomers thereof, or pharmaceutically acceptable salts, hydrates or solvates thereof, or a pharmaceutical composition of claim 7, for the preparation of a pharmaceutical composition for preventing and/or treating diseases related to the activity or expression of PD-1/PD-L1.

10. A PD-1/PD-L1 inhibitor, comprises the compound of claim 1, or stereoisomers or tautomers thereof, or pharmaceutically acceptable salts, hydrates or solvates thereof.
